# FASCICULE DE BREVET EUROPEEN

(11) **EP 1 468 003 B1**
(45) Date de publication et mention de la délivrance du brevet: **24.08.2005**
(21) Numéro de dépôt: 03717341.6
(22) Date de dépôt: 16.01.2003
(51) Int. Cl.: C07F 9/30, C07F 9/572, C07F 9/32, A61K 31/675, A61P 9/00

(54) **DERIVES DE PSEUDO-PEPTIDES PHOSPHINIQUES**
PHOSPHINSÄURE PSEUDO-PEPTIDDERIVATE
PHOSPHINIC PSEUDO-PEPTIDE DERIVATIVES

(30) Priorité: 18.01.2002 FR 0200599
(43) Date de publication de la demande: 20.10.2004
(73) Titulaire: COMMISSARIAT A L'ENERGIE ATOMIQUE, 75752 Paris (FR)
(72) Inventeur: COTTON, Jo, F-91400 Orsay (FR); GEORGIADIS, Dimitri, Athènes (GR); DIVE, Vincent, F-91120 Palaiseau (FR)
(74) Mandataire: Poulin, Gérard
(86) Numéro de dépôt international: PCT/FR2003/000129
(87) Numéro de publication internationale: WO 2003/062247

(56) Documents cités:
- EP-A- 0 361 341
- FR-A- 2 781 230
- US-A- 5 476 847
- DEMANGE L ET AL: "Synthesis of phosphinic alanyl-proline surrogates Ala psi (PO2R-CH) Pro as potential inhibitors of the human cyclophilin hCyp-18" TETRAHEDRON LETTERS, ELSEVIER SCIENCE PUBLISHERS, AMSTERDAM, NL, vol. 42, no. 36, 3 septembre 2001 (2001-09-03), pages 6295-6297, XP004302932 ISSN: 0040-4039

## Description

### Domaine technique

La présente invention a pour objet l'utilisation de dérivés de pseudo-peptides phosphiniques pour la préparation d'un médicament pour inhiber sélectivement le site actif C-terminal de l'enzyme de conversion de l'angiotensine I humaine (ACE), c'est-à-dire sans affecter le site actif N-terminal de l'ACE.

Un tel médicament est susceptible d'être utilisé dans la prévention et le traitement de différentes pathologies cardiovasculaires chez l'homme.

La présente invention a également pour objet des dérivés de pseudo-peptides phosphiniques nouveaux, des compositions pharmaceutiques les contenant, ainsi que des procédés de préparation desdits dérivés de pseudo-peptides phosphiniques.

### Etat de la technique antérieure

L'enzyme de conversion de l'angiotensine I (ACE) est un acteur central de la régulation de la pression artérielle et de l'homéostase des différentes fonctions physiologiques du tissu cardiovasculaire. Ces actions semblent en partie dépendre :
- de la maturation d'un puissant vasoconstricteur, l'angiotensine II, par clivage de l'extrémité C-terminale de l'angiotensine I, peptide inactif, par l'ACE, et
- de la dégradation par l'ACE d'un puissant vasodilatateur, la bradykinine.

Ces actions sont illustrées ci-dessous.

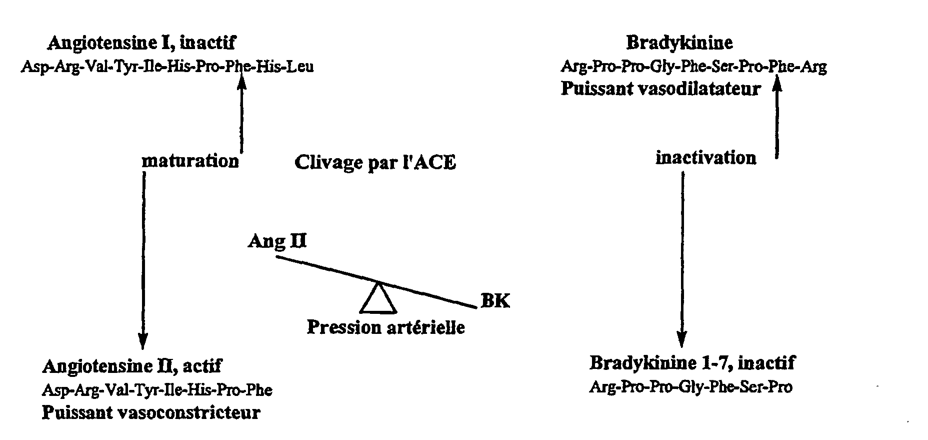

L'hypertension artérielle, mais aussi les maladies du tissu cardiaque, résultent d'une dérégulation des différentes hormones vaso-constrictives. Rétablir l'équilibre entre agents vasoconstricteurs et vasodilatateurs, au profit de ces derniers, est un des principaux objectifs thérapeutiques des médicaments utilisés en clinique humaine pour remédier à l'hypertension artérielle et aux maladies du tissu cardiaque. On comprend, ainsi, comment l'inhibition de l'ACE peut participer à ces objectifs, en empêchant la formation de l'angiotensine II et en potentialisant la bradykinine. Les inhibiteurs de l'ACE sont utilisés en clinique humaine, non seulement pour réduire l'hypertension artérielle, mais aussi pour préserver les fonctions du tissu cardiaque, comme décrit dans les références [1] et [2].

Le clonage de l'ACE, puis la détermination de sa structure primaire, ont montré de façon surprenante la présence de deux sites actifs dans cet enzyme, comme décrit dans la référence [3]. Par mutagénèse dirigée, il a pu être prouvé que ces deux sites actifs sont parfaitement fonctionnels, c'est-à-dire capables de cliver des substrats physiologiques de l'ACE, tels que l'angiotensine I et la bradykinine (références [4] et [5]).

Malgré tous les travaux réalisés depuis plus de vingt ans sur l'ACE, on ne sait toujours pas si la présence de deux sites actifs dans l'ACE de mammifères, résultant d'une duplication d'un gène ancestral, correspond à un rôle fonctionnel particulier. Cependant la découverte récente, qu'in vivo, chez l'homme, le peptide Ac-SDKP (N-acétyl Ser-Asp-Lys-Pro) est essentiellement clivé par le site actif N-terminal de l'ACE, milite en faveur d'un rôle fonctionnel distinct pour chacun des sites actifs de l'ACE (référence [6]).

Ces considérations ont conduit les chercheurs à tenter de développer des inhibiteurs capables de discriminer de façon hautement sélective les deux sites actifs de l'ACE, afin de disposer d'outils pouvant permettre d'établir *in vivo* le rôle fonctionnel de chacun des sites de l'ACE.

A cet égard, il est important de souligner que tous les inhibiteurs utilisés à ce jour en clinique sont des inhibiteurs mixtes de l'ACE, c'est-à-dire qui bloquent simultanément les deux sites actifs de l'ACE.

On a mis au point récemment le premier inhibiteur bloquant de façon sélective le site N-terminal de l'ACE, le RXP407 qui est un pseudo-peptide phosphinique (références [7], [8] et [21]). Cet inhibiteur, non métabolisé chez le rat et la souris, est, par ailleurs, capable d'inhiber la dégradation du peptide N-acétyl Ser-Asp-Lys-Pro (Ac-SDKP) *in vivo* chez la souris (référence [9]). Ainsi, l'injection de RXP407, en bloquant le site N-terminal de l'ACE, empêcherait *in vivo* la dégradation de l'Ac-SDKP.

Cet inhibiteur fait l'objet d'une étude préclinique chez cet animal, visant à démontrer son utilité pour protéger le tissu hématopoïétique lors de traitements de chimiothérapie.

Par contre, aucun inhibiteur capable de discriminer les deux sites actifs de l'ACÈ en interagissant essentiellement avec le site C-terminal de l'ACE n'a été décrit à ce jour. Pourtant, il serait souhaitable de disposer de tels inhibiteurs. En effet, outre leur intérêt en recherche expérimentale et clinique, ils présenteraient l'avantage majeur, par rapport aux inhibiteurs mixtes classiques de l'ACE, de ne pas interférer avec les fonctions physiologiques liées à l'activité du site N-terminal de l'ACE, comme le métabolisme du peptide Ac-SDKP.

Il a été démontré que les peptides phosphiniques représentent une famille générique de composés capables d'inhiber, de façon très puissante, les métallopeptidases à zinc, famille de peptidases à laquelle appartient l'ACE, comme on peut le voir dans les références [9) à [16]. Dans ces composés, le rôle du groupe PO₂⁻ est d'interagir avec l'atome de zinc situé dans le site actif de ces enzymes.

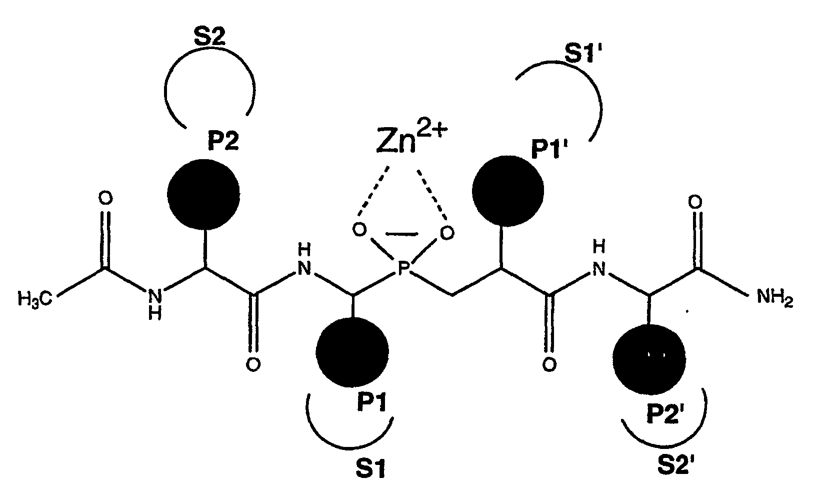

Outre la présence du groupe PO₂⁻, la nature chimique des résidus P2, P1, P1' et P2' joue un rôle déterminant pour assurer la sélectivité des interactions entre un peptide phosphinique particulier et une métallopeptidase à zinc donnée (voir références [8], [12] et [13]). Ainsi, la présence de résidus bien particuliers dans les positions P2, P1, P1' et P2' permet d'obtenir des inhibiteurs sélectifs, n'inhibant que certaines métallopeptidases à zinc. Une telle sélectivité peut être un facteur essentiel dans le cadre d'une utilisation *in vivo* de ces inhibiteurs. En effet, on estime que la toxicité *in vivo* de certains inhibiteurs est en grande partie due à leur manque de sélectivité pour une cible donnée.

Selon ces principes, la recherche d'inhibiteurs capables de bloquer sélectivement le site C-terminal de l'ACE a consisté à identifier dans la famille des composés phosphiniques, des résidus particuliers, situés dans les positions P1, P1' et P2', conférant aux inhibiteurs une capacité à interagir sélectivement avec le site C-terminal de l'ACE.

Des pseudo-peptides phosphiniques ont, par ailleurs, été proposés en tant qu'inhibiteurs de la cyclophiline hCyp-18 humaine, de la protéase du VIH et de l'enzyme de conversion des endothélines dans les réferences [22] à [24].

### Exposé de l'invention

Ces recherches ont conduit à découvrir que la présence d'un résidu pseudo-proline dans des pseudo-peptides phosphiniques constitue un élément essentiel pour obtenir des inhibiteurs sélectifs du site C-terminal de l'ACE.

Aussi, la présente invention a pour objet l'utilisation d'au moins un dérivé de pseudo-peptide phosphinique comportant la séquence d'acides aminés de formule (I) suivante : 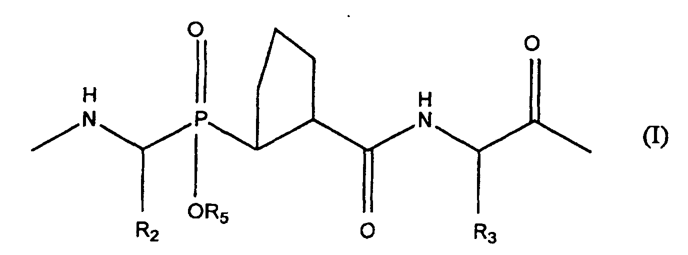 dans laquelle :
- R₂ et R₃, qui sont identiques ou différents, représentent la chaîne latérale d'un acide aminé naturel ou non naturel, l'ensemble : 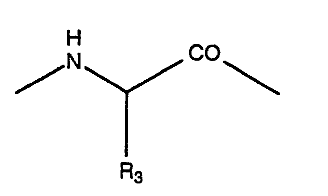 pouvant aussi former le résidu Pro (proline), et
- R₅ représente un atome d'hydrogène, un contre-ion acceptable du point de vue pharmacologique, ou un groupe capable de former un ester phosphinique hydrolysable *in vivo* ;
pour la fabrication d'un médicament capable d'inhiber sélectivement le site C-terminal de l'enzyme de conversion de l'angiotensine I.

Dans cette séquence, le groupe PO₂ peut être sous forme PO₂⁻ en étant associé à un atome d'hydrogène ou à un contre-ion acceptable du point de vue pharmacologique, par exemple K⁺, Na⁺, NH₄⁺ ou tout autre ion métallique ou non métallique acceptable du point de vue pharmacologique. La nature du contre-ion n'a aucune importance car dans l'eau les groupements chargés sont dissociés.

Le groupe PO₂ peut aussi être sous forme d'ester phosphinique hydrolysable *in vivo*. Dans ce cas, le pseudo-peptide est du type pro-drogue et, après hydrolyse *in vivo* de l'ester, il génère la forme active du pseudo-peptide.

Des groupes de ce type utilisables pour R₅ sont décrits en particulier dans la référence [20].

A titre d'exemples de tels groupes, on peut citer les groupes répondant aux formules suivantes :

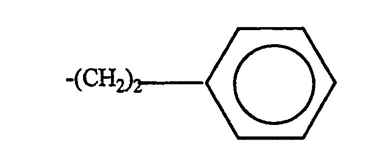

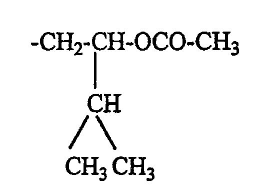

-CH₂)₃-S-CO-CH₃

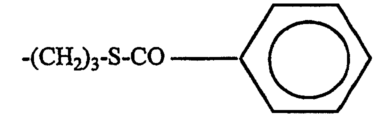

-(CH₂)₂-O-CO-tBu

-(CH₂)₃-S-CO-tBu

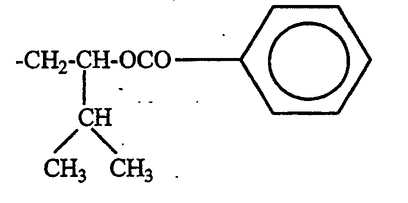

Dans ces formules, t-Bu représente le groupe tert-butyle.

Selon un mode particulier de mise en oeuvre de l'invention, le dérivé de pseudo-peptide phosphinique répond à la formule (II) suivante : 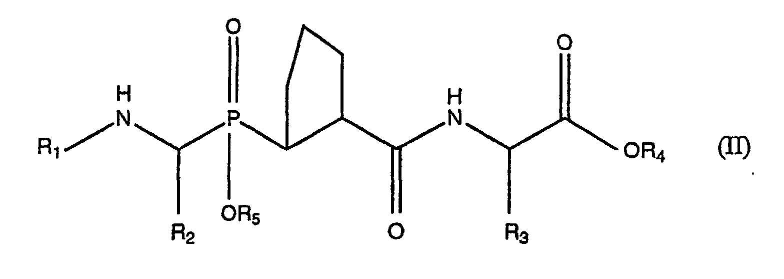 dans laquelle :
- R₁ représente un groupe protecteur d'une fonction amine, ou un acide aminé ou un peptide protégé par un groupe protecteur d'une fonction amine,
- R₂ et R₃, qui peuvent être identiques ou différents, représentent la chaîne latérale d'un acide aminé naturel ou non naturel, l'ensemble : 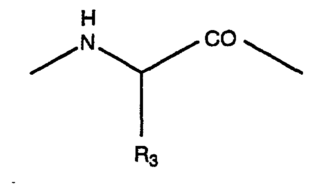 pouvant aussi former le résidu Pro,
- R₄ représente un atome d'hydrogène ou un contre-ion acceptable du point de vue pharmacologique, et
- R₅ est tel que défini ci-dessus.

Dans les formules données ci-dessus, R₂ et R₃ représentent la chaîne latérale d'un acide aminé naturel ou non naturel, par exemple un pseudo-acide aminé.

Les acides aminés naturels peuvent être choisis parmi l'alanine, l'arginine, l'asparagine, l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la tyrosine, la valine, la nitrophénylalanine, l'homoarginine, la thiozolidine et la déshydropoline.

Un pseudo-acide aminé peut être défini comme un acide aminé dans lequel la fonction amino ou carbonyle a été remplacée par un autre groupement chimique.

Dans la formule (II) ci-avant mentionnée, le groupe R₁ peut être un groupe protecteur usuel d'une fonction amine en chimie peptidique. De tels groupes protecteurs sont bien connus de l'homme du métier et sont illustrés, par exemple, dans l'ouvrage intitulé "Protective groups in Organic Synthesis, Second Edition, T.W. Greene et P.G.M. Wuts, John Wiley & Sons, Inc., pages 309-315 [17]. A titre d'exemples de tels groupes utilisables dans l'invention, on peut citer les groupes acétyle et benzyloxycarbonyle.

R₁ peut aussi représenter un acide aminé naturel ou non naturel ou un peptide dont la fonction amine terminale est protégée par un groupe protecteur usuel tel que ceux décrits ci-dessus.

Selon l'invention, comme on le verra ci-après, la présence du résidu pseudo-proline est essentielle pour obtenir la sélectivité vis-à-vis du site actif C-terminal de l'ACE, mais la nature des chaînes latérales présentes en R₂ et R₃ joue aussi un rôle important dans la sélectivité des interactions des dérivés utilisés selon l'invention avec les sites N et C-terminaux de l'ACE.

De bons résultats en ce qui concerne l'inhibition du site C-terminal de l'ACE, ont été obtenus avec des pseudo-peptides dans lesquels le groupe R₂ représente le groupe benzyle, méthyle ou phénéthyle, soit la chaîne latérale de la phénylalanine, de l'alanine et de l'homo-phénylalanine.

Pour R₃, on a obtenu de bons résultats lorsque R₃ représente la chaîne latérale de l'alanine, de l'arginine ou du tryptophane, ou lorsque l'ensemble -NH-CH(R₃)-CO- représente le résidu Pro.

Généralement, R₄ et R₅ représentent un atome d'hydrogène.

Selon un mode préféré de mise en oeuvre de l'invention, le dérivé de pseudo-peptide phosphinique répond à la formule suivante :

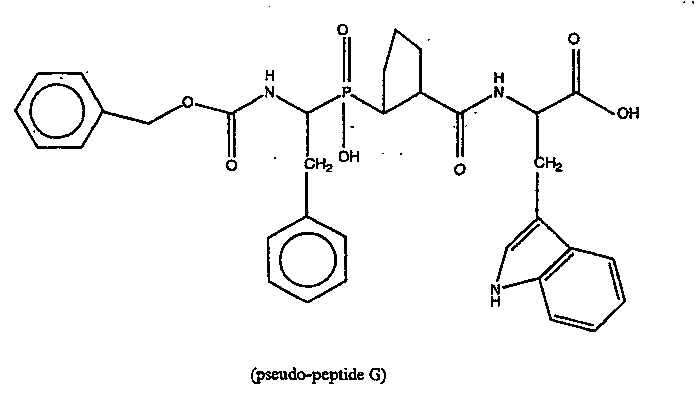

Les dérivés de pseudo-peptides phosphiniques susceptibles d'être utilisés conformément à l'invention se sont révélés être capables d'inhiber sélectivement le site actif C-terminal de l'enzyme de conversion de l'angiotensine I et, donc, de contrôler *in vivo* les taux physiologiques de l'angiotensine II - laquelle joue un rôle central dans le contrôle de la pression artérielle ainsi que de l'homéostase des fonctions cardiovasculaires chez l'homme - sans interférer pour autant sur le métabolisme de la bradykinine, ni sur celui du peptide Ac-SDKP.

Leur utilisation en tant que principes actifs dans un médicament est donc susceptible de trouver de nombreuses applications dans la prévention et le traitement des pathologies cardiovasculaires humaines, et notamment des pathologies dans lesquelles la bradykinine interviendrait peu comme la prévention de l'athérosclérose.

Parmi les dérivés de pseudo-peptides phosphiniques dont l'utilisation est envisagée selon l'invention, il en est qui n'ont jamais été décrits dans la littérature.

Aussi, l'invention a-t-elle également pour objet un dérivé de pseudo-peptide phosphinique comportant la séquence d'acides aminés de formule (I) ci-avant mentionnée, dans laquelle :
- R₂ représente la chaîne latérale d'un acide aminé naturel ou non naturel,
- l'ensemble : 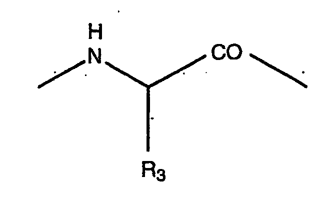 forme le résidu Pro : 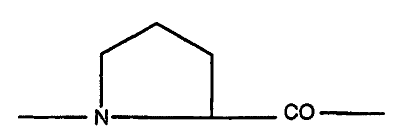 et
- R₅ représente un atome d'hydrogène, un contre-ion acceptable du point de vue pharmacologique, ou un groupe capable de former un ester phosphinique hydrolysable *in vivo*.

Parmi ces dérivés, on préfère notamment ceux qui répondent à la formule (II) ci-avant mentionnée, dans laquelle :
- R₁ représente un groupe protecteur d'une fonction amine, ou un acide aminé ou un peptide protégé par un groupe protecteur d'une fonction amine,
- R₂ représente la chaîne latérale d'un acide aminé naturel ou non naturel,
- l'ensemble : 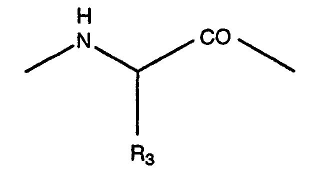 forme le résidu Pro : 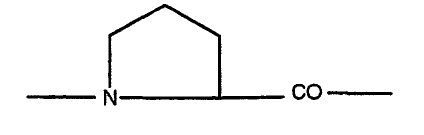
- R₄ représente un atome d'hydrogène ou un contre-ion acceptable du point de vue pharmacologique,
- R₅ représente un atome d'hydrogène, un contre-ion acceptable du point de vue pharmacologique, ou un groupe capable de former un ester phosphinique hydrolysable *in vivo*.

Plus particulièrement, on préfère le dérivé de pseudo-peptide phosphinique de formule :

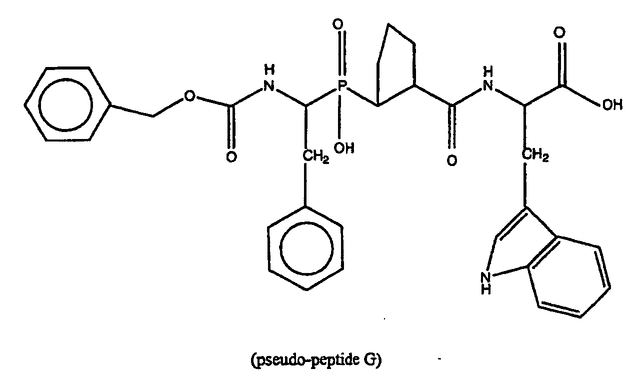

L'invention a encore pour objet une composition pharmaceutique qui comprend au moins un dérivé de pseudo-peptide phosphinique répondant à la formule (II) ci-avant mentionnée, dans laquelle :
- R₂ représente la chaîne latérale d'un acide aminé naturel ou non naturel,
- l'ensemble : 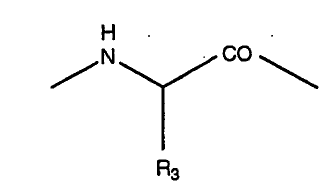 forme le résidu Pro : 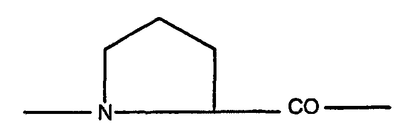 et
- R₅ représente un atome d'hydrogène, un contre-ion acceptable du point de vue pharmacologique, ou un groupe capable de former un ester phosphinique hydrolysable *in vivo.*

Dans cette composition, le dérivé pseudo-peptidique phosphinique répond, de préférence, à la formule :

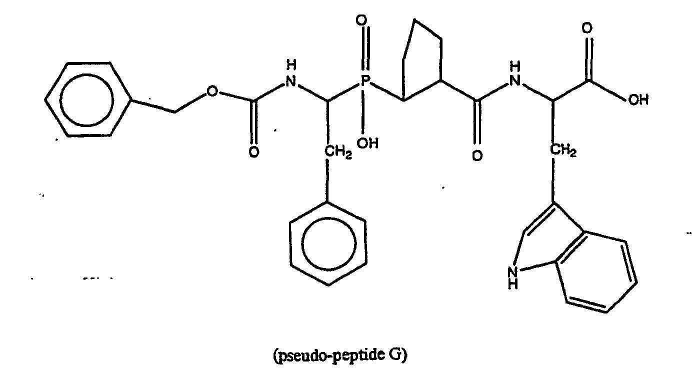

Eu égard à ce qui précède, une telle composition pharmaceutique est notamment susceptible de trouver de multiples applications dans la prévention et le traitement de différentes pathologies cardiovasculaires chez l'homme.

Les dérivés de pseudo-peptides phosphiniques répondant à la formule (II) ci-avant mentionnée, dans laquelle R₄ et R₅ représentent un atome d'hydrogène, peuvent être préparés par un procédé comprenant les étapes suivantes :
1) faire réagir un composé de formule (III) : 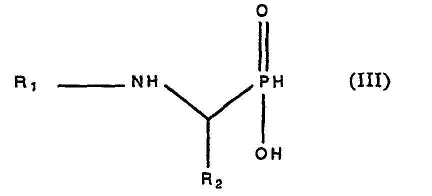 dans laquelle R₁ et R₂ sont tels que définis ci-dessus avec le composé de formule (IV) : 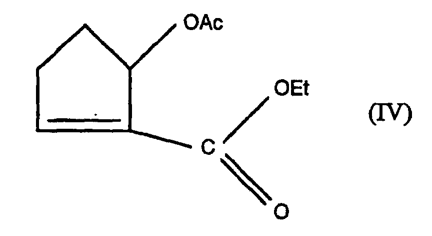 dans laquelle Ac représente le groupe acétyle et Et représente le groupe éthyle, pour obtenir le composé de formule (V) : 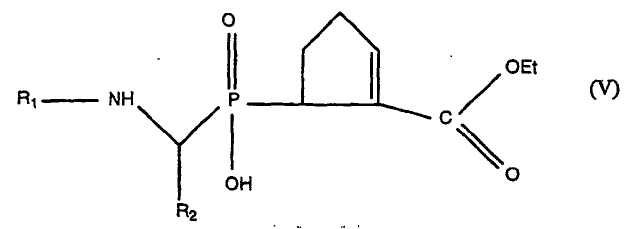
2) transformer le composé (V) en composé (VI) par réaction du composé (V) avec du borohydrure de sodium : 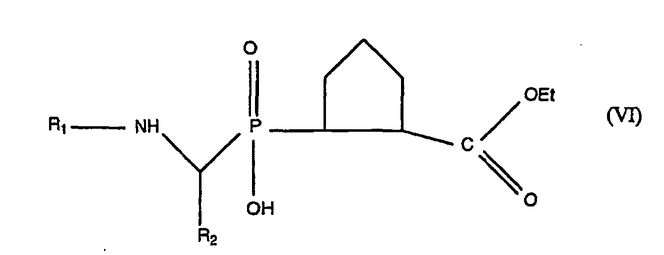
3) protéger le groupe hydroxyle du composé (VI) par un groupe adamantyle Ad pour obtenir le composé de formule (VII) : 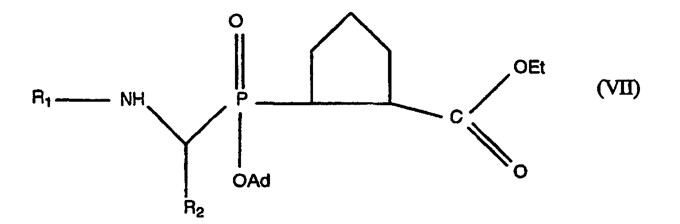
4) saponifier le composé (VII) pour obtenir le composé de formule (VIII) : 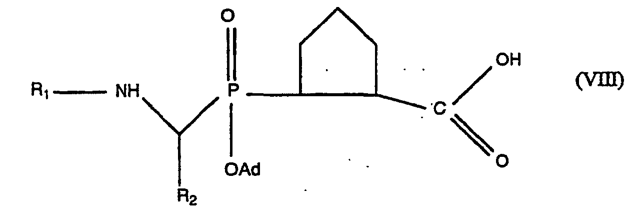
5) coupler le composé de formule (VIII) avec l'acide aminé de formule (IX) ou (X) : 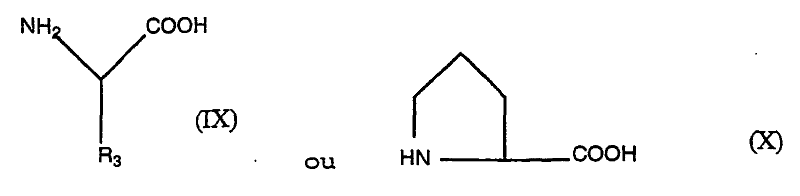 dans laquelle R₃ est tel que défini ci-dessus, et
6) éliminer le groupe protecteur Ad.

Selon ce procédé, on synthétise tout d'abord le bloc phosphinique de formule (VIII) comprenant la pseudo-proline, et on effectue ensuite un couplage peptidique de ce bloc phosphinique avec l'acide aminé voulu.

Avantageusement, l'étape 5) de couplage peptidique est réalisée par synthèse peptidique sur phase solide en utilisant comme phase solide une résine substituée par l'acide aminé de formule (IX) ou (X), dont l'extrémité N-terminale aura été préalablement protégée par un groupe Fmoc (fluorényl méthoxy carbonyle).

Si nécessaire, on peut ensuite estérifier ou salifier la fonction phosphinique du pseudo-peptide de formule (II) dans laquelle R₅ représente un atome d'hydrogène, en le faisant réagir avec des réactifs appropriés.

L'estérification peut être obtenue par couplage avec un alcool de formule R₅OH dans laquelle R₅ représente un groupe capable de former un ester phosphinique hydrolysable *in vivo*, en utilisant par exemple le procédé décrit dans la référence [20] (méthode A).

On peut aussi réaliser l'estérification par réaction avec un halogénure de formule R₅X dans laquelle R₅ représente le groupe capable de former un ester phosphinique hydrolysable *in vivo*. Cette réaction peut être effectuée dans des conditions alcalines en utilisant le procédé décrit dans la référence [20] (méthode B).

Avant d'effectuer cette estérification, on protège la(les) fonction(s) acide(s) carboxylique(s) du pseudo-peptide par des groupes protecteurs appropriés que l'on élimine ensuite en utilisant des techniques classiques.

Lorsqu'on veut salifier la fonction phosphinique du pseudo-peptide de formule (II) dans laquelle R₅ est un atome d'hydrogène, pour remplacer cet atome d'hydrogène par un contre-ion pharmaceutiquement acceptable, on fait réagir le pseudo-peptide avec une base appropriée contenant ce contre-ion, par exemple NaOH, KOH, NH₄OH.

On peut utiliser la même technique pour salifier le groupe carboxylique terminal du pseudo-peptide afin de remplacer l'atome d'hydrogène par un contre-ion pharmaceutiquement acceptable.

D'autres caractéristiques de l'invention apparaîtront mieux à la lecture du complément de description qui suit, qui se rapporte à des exemples de réalisation de dérivés de pseudo-peptides conformes à l'invention et de démonstration de leurs propriétés, en référence aux dessins annexés.

Bien entendu, ce complément de description est donné à titre illustratif et non limitatif de l'objet de l'invention.

### Brève description des dessins

La figure 1 illustre la synthèse de synthons utiles pour la préparation des pseudo-peptides phosphiniques conformes à l'invention.

La figure 2 illustre le chromatogramme obtenu lors de la purification du pseudo-peptide G par chromatographie liquide à haute performance (HPLC).

La figure 3 illustre les effets *in vivo* du pseudo-peptide G sur le clivage de l'angiotensine I en angiotensine II.

La figure 4 illustre les effets *in vivo* du pseudo-peptide G sur le clivage de la bradykinine.

La figure 5 illustre les effets *in vivo* du pseudo-peptide G et du RPX407 (inhibiteur sélectif du site N-terminal de l'ACE) sur le clivage du peptide Ac-SDKP.

### Exposé détaillé des modes de réalisation

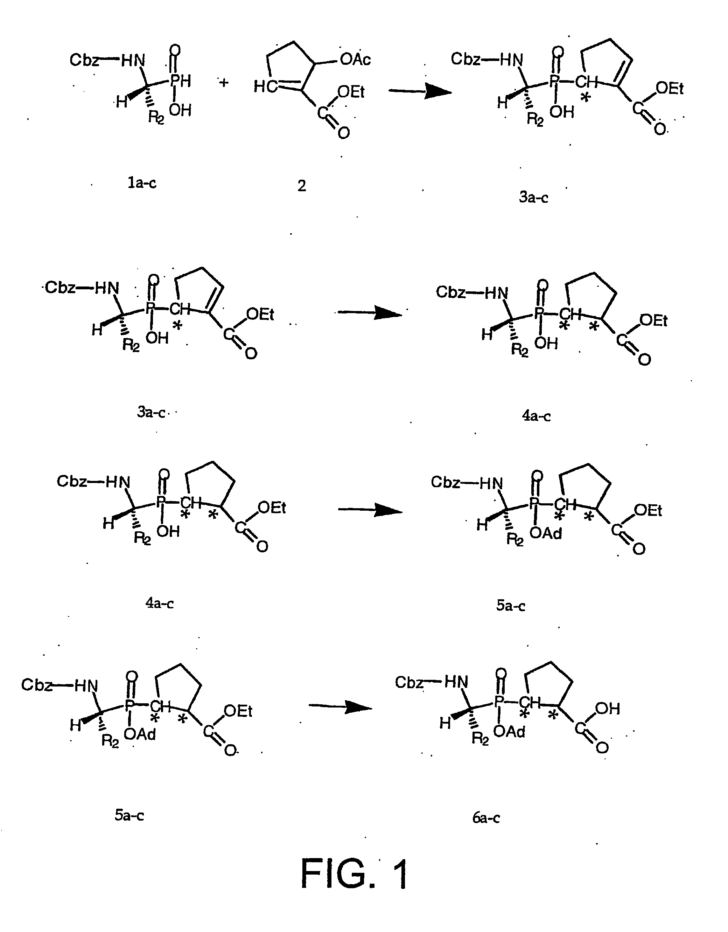

La synthèse des pseudo-peptides phosphiniques a été effectuée en suivant le schéma de synthèse décrit sur la figure 1.

Cette figure représente les étapes du procédé aboutissant aux synthons de formule (VIII) : 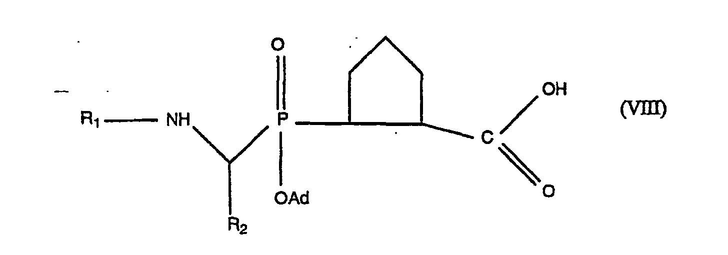 dans laquelle :
- R₁ représente le groupe benzyloxycarbonyle (Cbz), et
- R₂ est le groupe phényle (composés 1a, 3a, 4a, 5a et 6a), le groupe phénéthyle (composés 1b, 3b, 4b, 5b et 6b) ou le groupe méthyle (composés 1c, 3c, 4c, 5c et 6c).

### Exemple 1 :préparation du composé 6a

### 1) Préparation du composé 1a

Ce dérivé d'acide amino-phosphinique est préparé selon la procédure décrite par Baylis [18], puis l'énantiomère de configuration R est obtenu par recristallisation en suivant le protocole rapporté dans Baylis [18].

### 2) Préparation du composé 2a

Ce composé est obtenu en suivant la procédure publiée par Villieras et al [19]. Le produit obtenu a été caractérisé par RMN :
¹H-RMN (250 MHz, CDCl₃) : 7,03 (t, 1H), 5,93 (m, 1H), 4,1 (m, 2H), 2,6 (m, 1H), 2,34 (m, 2H), 1,95 (s, 3H), 1,82 (m, 1H), 1,18 (t, 3H).

### 3) Préparation du composé 3a

Un mélange du composé 1a (3,2 g, 10 mmol) et d'hexaméthyldisilazane (10,5 mL, 50 mmol) sous flux d'argon est chauffé à 110°C durant 3h. A cette température, le composé 2 (5,5 g, 12 mmol) est ajouté et cette solution est mise sous agitation pour 4h à 90°C. A cette solution refroidie à 70°C, 10 mL d'éthanol EtOH absolu sont ajoutés, goutte à goutte, le mélange étant agité à 70°C pendant 30 min. Après évaporation des solvants, le résidu est solubilisé dans 5% NaHCO₃ (10 mL) et 5 mL d'hexane. Après 3 extractions avec de l'acétate d'éthyle AcOEt (3 x 5 mL), le produit brut est obtenu après évaporation du solvant. Une purification sur colonne de silice, utilisant comme phase mobile un mélange chloroforme/méthanol/acide acétique (7/0,3/0,3) permet d'obtenir 4 g de composé 3a pur, sous forme d'un solide blanc (rendement de 89%).

La caractérisation RMN de ce produit est basée sur des expériences COSY, TOCSY et HMQC :
¹H-RMN (250 MHz, CDCl₃) : 1,27 (t, ³J_{HH}= 7,1Hz, 3H, CH2CH3), 1,95-3,00 (m, 5H, PCH(CH₂)₂, PhCHH), 3,13-3,45 (m, 2H, PCB, PhCHH), 4,02-4,31(m, 2H, CH₂CH₃), 4,34-4,63(m, 1H, PCH), 4,78-5,05(m, 2H, OCH₂Ph), 5,71(d, 1H, NH, ³J_{HH}=11,3Hz, I), 5,78 (d, 1H, NH, ³J_{HH}=10,8Hz, II), 6,91-6,99 (d, 1H, C=CH, I/II), 7,02-7,34(m, 10H, aryl). ¹³C-RMN (62MHz, CDCl₃) : 14,2/14,2 (CH2-CH3), 26,2/26,5 (PCHCH₂), 32,7/32,8 (PCHCH₂CH₂), 34,5 (CH₂Ph), 41,8 (d, ¹J_{PC}=87,7Hz, PCHC, I), 43,1(d, ¹J_{PC}=87,3Hz, PCHC, II), 50,5(d, ¹J_{PC}=99,7Hz, PCHN, I), 50,5(d, ¹J_{PC}=100,5Hz, PCHN, II), 61,1/61,2 (CH₂CH₃), 66,8 (OCH₂Ph), 126,6, 127,7, 127,8, 127,9, 127,9, 128,4, 128,4, 129,4, 132,2, 132,3, 132,9, 136,5, 136,6, 137,1, 137,3, (aryles), 148,1 (d, ²J_{PC}=8,7Hz, =CC0, I), 148,1 (d, ²J_{PC}=9Hz, =CC0, II), 156,1 (d, ²J_{PC}=5,5Hz,CONH, I), 156,2((d, ²J_{PC}=5,7Hz,CONH, II), 165,3 (d, ²J_{PC}=2,7Hz, COOEt).
³¹P-RMN (100MHz, CDCl₃) : 46,89, 48,13.

Les mentions I et II correspondent aux différents diastéréoisomères.

| Analyse élémentaire : | | |
|---|---|---|
| Valeurs théoriques : | | |
| C : 61,80%, | H : 6,27%, | N : 3,00% |

| Valeurs expérimentales : | | |
|---|---|---|
| C : 61,89%, | H : 6,23%, | N : 2,98% |

### 4) Préparation du composé 4a

Le composé 3a (1,4 g, 3,06 mmol) et du NiCl₂.6H₂O (1,09 g, 9,2 mmol) sont solubilisés dans un mélange THF (12,4 mL) /méthanol (7,7 mL). A cette solution est ajouté par portions du NaBH₄ (0,58 g, 15,4 mmol) pendant 30 min, à -30°C. Ce mélange reste sous agitation pendant 10 min, à -30°C. Les solvants sont évaporés et le produit est extrait dans un mélange AcOEt (25 mL) et HCl 1N (20 mL, pH 1).

La phase organique est prélevée et lavée à l'eau (10 mL), puis séchée avec du Na₂SO₄. Après évaporation des solvants, le produit est purifié sur colonne de silice avec une phase mobile chloroforme/méthanol/acide acétique (7/0,3/0,3).On obtient 1,28 g du composé 4a (rendement de 91%).

L'analyse par spectrométrie de masse mode négatif (masse observée MH⁻ = 458,48, masse attendue = 459,47) est en accord avec la structure chimique du composé 4a.

| Analyse élémentaire : | | |
|---|---|---|
| Valeurs théoriques : | | |
| C : 61,78%, | H : 6,63%, | N : 3,00% |

| Valeurs expérimentales : | | |
|---|---|---|
| C : 61,98%, | H : 6,31%, | N : 3,08% |

### 5) Préparation du composé 5a

L'adamantylation du composé 4a est réalisée en suivant le protocole décrit par Yiotakis et al [14].

A une solution du composé 4a (1,03 g, 2,24 mmol) dans le chloroforme, 1-Adamantyl-Br (538 mg, 2,5 mmol) et Ag₂O (577 mg), répartis en 5 portions égales, sont ajoutés pendant 1h. Après 2h, 0,5 eq de AdBr et 0, 5 eq de Ag₂O ont été rajoutés et le mélange porté au reflux pendant 10h. Après évaporation des solvants, le produit brut est purifié sur colonne de silice en utilisant comme phase mobile un mélange chïoroforme/isopropanol (9,8/0,2). Le composé 5a est obtenu sous forme pure avec un rendement de 96% (1,27 g).

Analyse par spectrométrie de masse, mode positif : masse observée MH⁺ = 594,21, masse attendue = 593,1.

| Analyse élémentaire : | | |
|---|---|---|
| Valeurs théoriques : | | |
| C : 67,76%, | H : 7,53%, | N : 2,32% |

| Valeurs expérimentales : | | |
|---|---|---|
| C : 67,49%, | H : 7,58%, | N : 2,24% |

### 6) Préparation du composé 6a

Après dilution du composé 5a (1,1 g, 1,85 mmol) dans le méthanol (20 mL), 2 mL de NaOH 4N sont ajoutés. Après 6h sous agitation, le suivi de la réaction par TLC confirme la saponification complète du produit de départ. Après évaporation du solvant, le produit est repris dans un mélange eau (10 mL), puis ajout de AcOEt (15 mL) et acidification à pH 1 avec HCl 1N. Le résidu est repris dans la phase organique et la procédure d'extraction répétée deux fois. Les phases organiques rassemblées sont séchées sur Na₂SO₄, puis les solvants évaporés. Le composé pur 6a est obtenu avec un rendement de 94% (0,98 g).

Analyse par spectrométrie de masse, mode positif : masse observée MH⁺ = 566,15, masse attendue = 565,26.

| Analyse élémentaire : | | |
|---|---|---|
| Valeurs théoriques : | | |
| C : 67,95%, | H : 7,13%, | N : 2,48% |

| Valeurs expérimentales : | | |
|---|---|---|
| C : 67,64%, | H : 7,30%, | N :2,40 % |

### Exemple 2 :Préparation du composé 6b

On suit le même mode opératoire que dans l'exemple 1 pour préparer le composé 6b en partant du composé 1b qui est préparé selon la procédure décrite dans [18].

L'analyse par spectrométrie de masse du composé 6b a donné les résultats suivants : masse attendue = 579,27, masse observée MH⁺ = 580,29.

### Exemple 3 : préparation du composé 6c

On suit le même mode opératoire que dans l'exemple 1 pour préparer le composé 6c en partant du composé 1c qui est préparé selon la procédure décrite dans [18].

L'analyse par spectrométrie de masse du composé 6c a donné les résultats suivants : masse attendue = 489,23, masse observée MH⁺ = 490,11.

### Exemple 4 : préparation du pseudo-peptide G de formule :

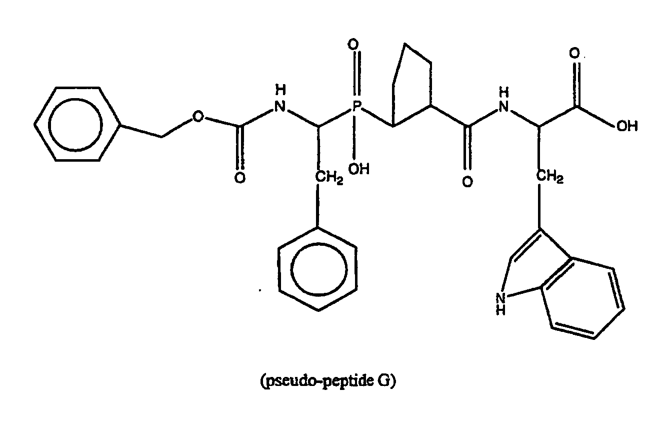

Ce pseudo-peptide a été synthétisé sur phase solide en utilisant un protocole standard de synthèse de peptides sur phase solide. La résine Wang substituée par un Fmoc-Trp (732 mg, 0,58 mmol) est suspendue dans la N-méthyl pyrrolidone NMP (5 mL) et agitée pendant 5 min. Après élimination de la NMP par filtration, 10 mL de pipéridine à 20% dans NMP sont ajoutés et le mélange est agité pendant 15 min. Après filtration, la résine est lavée avec les solvants suivants : NMP (7x10 mL), CH₂Cl₂ (3x10 mL) et Et₂O (2x10 mL). Sont ajoutés alors dans le réacteur, NMP 2ml, diisopropopyl éthylamine DIEA (749 mg, 5,76 mmol) et le composé 6a (360mg, 0,64 mmol) dilué dans la NMP (2 mL) et 2-(1H)benzotriazol-1-yl)-1,1,3,3-tétraméthyluronium hexafluorophosphate HBTU (730 mg, 1,92 mmol, dilué dans NMP 3 mL). Le mélange est laissé sous agitation pendant 24h. Après filtration, la résine est lavée avec la NMP (4x7 mL), CH₂Cl₂ (5x7 mL). Puis, une solution d'acide trifluoroacétique TFA/CH₂Cl₂/H₂O/triisopropylsilane (90/7,5/1,25/1,25) est ajoutée dans le réacteur et le mélange mis sous agitation pendant 3h (phase de déprotection). Après filtration, le filtrat contenant le pseudo-peptide G est récupéré, le solvant est évaporé et le produit est solubilisé dans H₂O. Après lyophilisation, le pseudo-peptide G est purifié par HPLC phase-inverse (colonne Vydac, C18, semi-préparative).

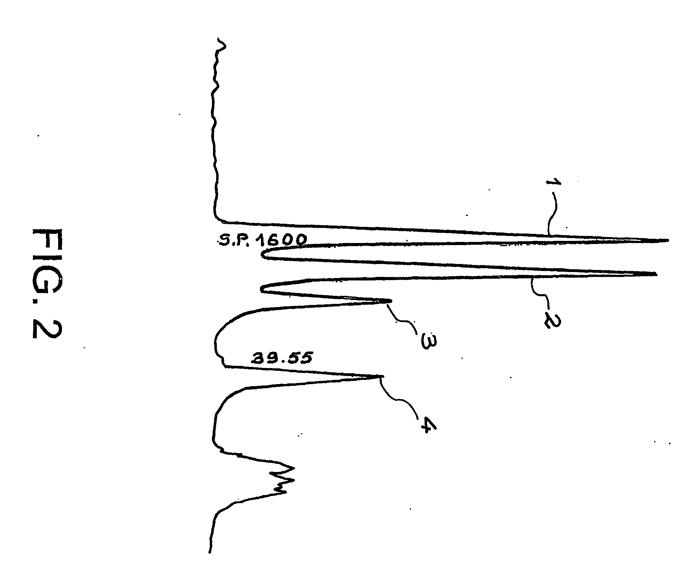

La figure 2 illustre le chromatogramme obtenu. Sur cette figure, on observe 4 pics correspondants aux 4 diastéréoisomères présents dans le pseudo-peptide G (ces quatre pics possèdent un spectre de masse identique, masse observée MH⁺= 618,23, masse attendue = 617,23). Seul le pic 1 montre un pouvoir inhibiteur vis-à-vis de l'ACE.

La caractérisation RMN du pseudo-peptide G, pic 1 HPLC, est basée sur des expériences COSY, TOCSY et HMQC :
¹H-RMN (250 MHz, DMSO) 4,93 (d, 2H, CH₂-O-Ph), 4,01 (m, 1H, CH-CH₂-Ph), 2,77(m, 1H, CH-CH₂-Ph), 3,08(m, 1H, CH-CH₂-Ph), 3,09 (α-pseudo-Proline), 1,77 (β-pseudo-Proline), 1,56 (γ-pseudo-Proline), 1,79(δ-pseudo-Proline), 2,49 (ε-pseudo-Proline), 4,50 (α-Trp), 3,13 (β,β-Trp), 7.6 NH, 8,3 NH, aryles 7,35-6,9.

### Exemples 5 à 7 : préparation des pseudo-peptides B, C et D de formules :

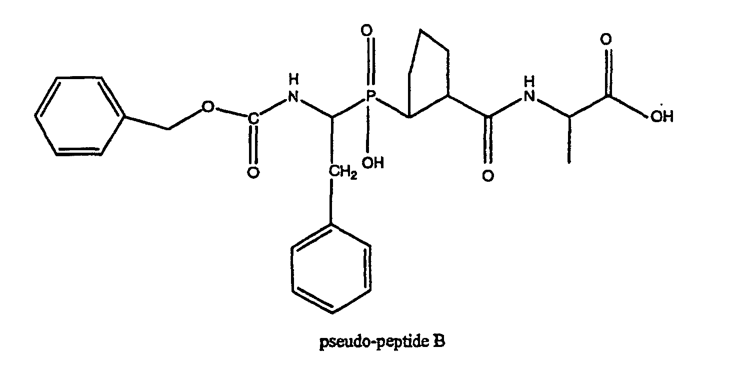

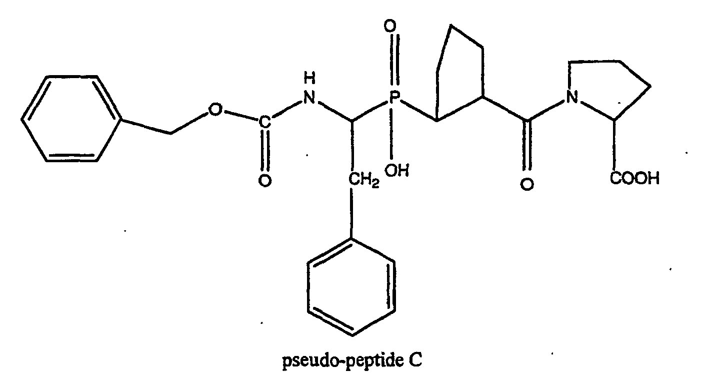

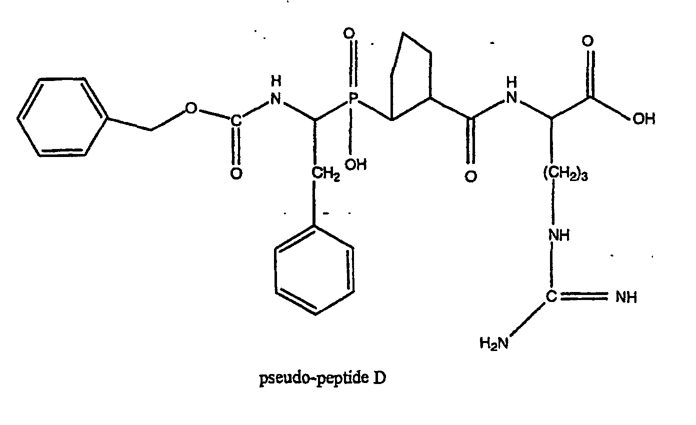

Les pseudo-peptides B, C et D ont été synthétisés en phase solide avec des résines Wang substituées par l'alanine (B), la proline (C) et l'arginine (D), en utilisant le protocole décrit pour la préparation du pseudo-peptide G. La purification de ces pseudo-peptides par HPLC a permis d'isoler des diastéréoisomères de ces pseudo-peptides capables d'inhiber l'ACE.

L'analyse des pseudo-peptides par spectrométrie de masse confirme la structure de ces pseudo-peptides.
Pseudo-peptide B : masse attendue 502,19 ; masse observée 503,21.
Pseudo-peptide C : masse attendue 528,53 ; masse observée 529,11.
Pseudo-peptide D : masse attendue 587,25 ; masse observée 587,24.

### Exemples 8 et 9 : préparation des pseudo-peptides E et F de formules :

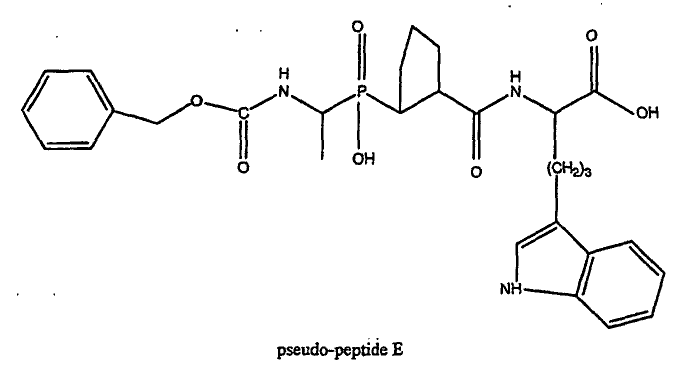

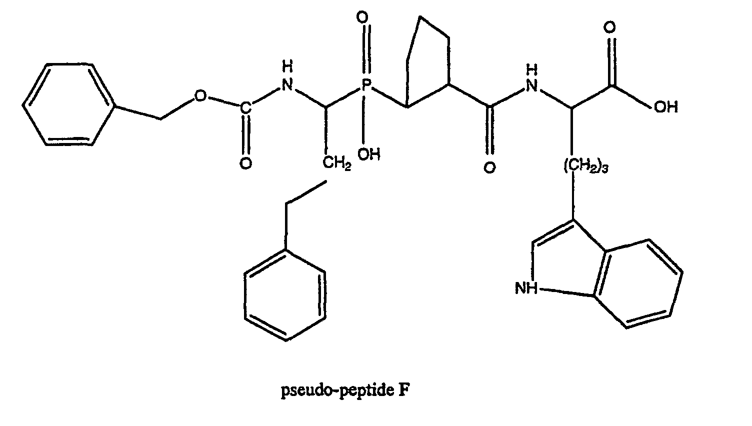

On obtient les pseudo-peptides E et F par synthèse en phase solide, en partant des composés 6b et 6c et en suivant le protocole décrit pour la préparation du pseudo-peptide G. Après purification par HPLC C18 mode inverse, la première fraction collectée pour chacun de ces pseudo-peptides s'est avérée capable d'inhiber l'ACE.

L'analyse par spectrométrie de masse a donné les résultats suivants :
PseLido-peptide E : masse attendue 541,20 ; masse observée 542,26.
Pseudo-peptide F : masse attendue 631,24 ; masse observée 632,26.

### Exemple 10 : détermination des constantes d'inhibition des pseudo-peptides A à G vis-à-vis des sites N et C terminaux de l'ACE

Pour cette détermination, on utilise de l'ACE recombinante humaine. Des courbes d'inhibition de l'ACE par les pseudo-peptides A à G sont obtenues en utilisant le substrat à fluorescence quenchée Mca-Ala : Mca-Ala-Ser-Asp-Lys-DpaOH (Mca : acide 7-méthoxy-coumarine-2-acétique ; DpaOH : N-3(2,4-dinitrophényl)L-2,3-diaminopropionyle).

Pour ces essais, on utilise la première fraction collectée lors de la purification de chacun des pseudo-peptides A à G par HPLC (pic 1 de la figure 2 pour le pseudo-peptide G).

A partir des profils d'inhibition obtenus avec ce substrat, pour chaque pseudo-peptide A à G, il est possible de déterminer les constantes Ki N et Ki C en suivant la procédure décrite dans Dive et al [8].

Les expériences d'inhibition ont été réalisées à 25°C, pH 6,8, 50 mM HEPES, 10 mM CaCl₂, 200 mM NaCl.

Les résultats obtenus sont donnés dans le tableau 1 annexé.

A titre comparatif, on a effectué le même essai avec le pseudo-peptide A ne comportant pas de résidu pseudo-proline. Les résultats obtenus sont également donnés dans le tableau 1. Ce pseudo-peptide a été préparé à partir du bloc phosphinique ZPhe[PO(OAd)-CH₂]AlaOH, décrit dans Yiotakis et al. [14], puis couplage de ce bloc à une résine Wang substituée par le résidu Fmoc-Ala.

L'étude des effets inhibiteurs des pseudo-peptides A à G sur l'ACE et la comparaison de leur affinité vis-à-vis du site N-terminal (Ki N) et du site C-terminal (Ki C) de l'ACE (tableau 1) permettent de tirer les conclusions suivantes.
**1°) Pseudo-peptides A et B :** pour ce qui concerne l'affinité vis-à-vis des deux sites actifs de l'ACE, la présence d'un résidu pseudo-proline apparaît bien moins favorable qu'un résidu pseudo-alanine. En revanche, la présence d'un résidu pseudo-proline en position P1' de ces pseudo-peptides phosphiniques donne accès à des inhibiteurs sélectifs du site C-terminal de l'ACE. Ce résultat démontre le rôle essentiel du résidu pseudo-proline pour contrôler la sélectivité des inhibiteurs vis-à-vis du site C-terminal de l'ACE.
**2°) Pseudo-peptides B, C, D et G :** Les modifications de la position P2' avec les résidus alanine, proline, arginine et tryptophane démontrent que la nature de la chaîne latérale dans cette position est aussi un facteur essentiel de la sélectivité. La présence du résidu proline (pseudo-peptide C) génère un inhibiteur puissant, mais peu sélectif des sites N et C de l'ACE. Par contre, la présence d'un résidu tryptophane permet d'obtenir un inhibiteur (pseudo-peptide G) extrêmement sélectif du site C-terminal de l'ACE.
**3°) Pseudo-peptides E et F :** La substitution d'un résidu pseudo-phénylalanine dans la position P1 des pseudo-peptides, par un résidu pseudo-alanine ou pseudo-homophénylalanine conduit à des pseudo-peptides moins puissants et moins sélectifs que le pseudo-peptide G. Ce dernier résultat démontre une importance moindre de la position P1 des inhibiteurs vis-à-vis de la sélectivité.

L'étude des pseudo-peptides A à G permet de conclure que, dans les pseudo-peptides de l'invention, chaque position P1, P1' et P2' contribue à la sélectivité des interactions. La présence dans le pseudo-peptide G des résidus pseudo-phénylalanine, pseudo-proline et tryptophane lui confère une sélectivité particulièrement prononcée.

### Exemple 11 : mise en évidence des propriétés in vivo du pseudo-peptide G

Une étude est réalisée *in vivo* en vue de vérifier l'aptitude du pseudo-peptide G à inhiber la conversion de l'angiotensine I en angiotensine II, et d'apprécier ses effets sur le clivage de la bradykinine d'une part, et du peptide Ac-SDKP d'autre part.

### 1) Protocole de l'étude

Cette étude est réalisée sur des lots de souris mâles C57BL6/J (Iffa Credo) pesant de 20 à 23 g chacune, chaque lot comprenant 6 souris.

Pour ce faire, les souris sont anesthésiées par une administration intrapéritonéale de pentobarbital de sodium (Sanofi) à la dose de 80 mg/kg de poids corporel. La carotide droite est isolée et un cathéter (PE10, 0,28x0,61, A-M Systems, Inc.) est mis en place dans cette artère pour permettre le prélèvement des échantillons sanguins, tandis qu'un autre cathéter (FEP, 0,12x0,67, Carnegie Medecin) est mis en place dans la jugulaire ipsilatérale pour permettre l'administration des substances. Pendant toute la durée de l'expérimentation, la température corporelle des souris est maintenue à 38°C.

Dans un premier temps, on administre aux souris d'un même lot, par perfusion de 30 min :
- soit 50 µL d'une solution isotonique (ajustée à pH 7) contenant une quantité de pseudo-peptide G correspondant à une dose de 0,9, 3, 10 ou 30 mg/kg de poids corporel,
- soit 50 µL de sérum physiologique,
- soit encore 50 µL d'une solution contenant une quantité de perindopril correspondant à une dose de 10 mg/kg de poids corporel, cette substance étant un puissant inhibiteur mixte de l'ACE (Servier).

Puis, on leur administre en bolus :
- soit un mélange comprenant 2 µg d'angiotensine I non marquée et 21 µCi de ³H-angiotensine I dans 50 µL de solution isotonique,
- soit un mélange comprenant 2 µg d'Ac-SDKP non marqué et 17 µCi de ³H-Ac-SDKP dans 50 µL de solution isotonique,
- soit un mélange comprenant 2 µg de bradykinine non marquée et 11 µCi de ³H-bradykinine dans 50 µL de solution isotonique.

Des prélèvements de sang artériel, d'environ 50 µL, sont réalisés 30, 60 et 90 sec après l'injection du mélange d'angiotensine I ou du mélange de bradykinine chez les souris ayant reçu l'un de ces mélanges, et 1, 5, 10 et 15 min après le début de l'injection du mélange d'Ac-SDKP chez les souris ayant recu ce dernier mélange. Dans tous les cas, le sang est recueilli dans des tubes en propylène préalablement pesés et contenant 40 µL d'eau, 10 µL de TFA à 80% et 1 µL d'héparine. Les quantités exactes de sang prélevé sont déterminées en repesant les tubes. Après addition de 195 µL d'eau distillée, ces tubes sont placés pendant 10 min dans un bain de glace et les échantillons sont centrifugés à 4°C pour obtenir des extraits plasmatiques.

L'analyse de ces extraits est réalisée par chromatographie liquide au moyen d'un système HPLC (PerkinElmer 200) lié à un détecteur de radioéléments (Z 500-4 cell, Berthold). Les séparations chromatographiques sont réalisées sur une colonne Kromasil C18 (AIT), par injection d'échantillons de 50 µL et en utilisant les phases mobiles et les gradients d'élution suivants :
- analyse de l'angiotensine I :
   phase mobile :
      solvant A : CH₃CN/H₂O/TFA (10/90/0,1)
      solvant B : CH₃CN/H₂O/TFA (90/10/0,1)
   gradients d'élution :
      0-30 min : 0-30% B
      30-35 min : 30-100% B
- analyse de la bradykinine :
   phase mobile :
      solvant A : CH₃CN/H₂O/TFA (10/90/0,1)
      solvant B : CH₃CN/H₂O/TFA (90/10/0,1)
   gradients d'élution :
      0-30 min : 0-25% B
      30-35 min : 25-100% B
- analyse de l'Ac-SDKP :
   phase mobile :
      solvant A : H₂O/TFA (100/0,1)
      solvant B : CH₃CN/H₂O/TFA (90/10/0,1)
   gradients d'élution :
      0-30 min : 0-30% B
      30-35 min : 30-100% B

Les pics élués sont identifiés par comparaison de leurs temps de rétention à ceux présentés par les susbtrats non marqués (angiotensine I, bradykinine et Ac-SDKP) et par les produits de clivage attendus (angiotensine II, BK(1-7) et BK(1-5)).

Les dosages sont réalisés par intégration de l'aire sous les pics correspondants du chromatogramme. Les valeurs ainsi obtenues sont normalisées en fonction du poids de sang prélevé chez chaque souris.

Les comparaisons statistiques des résultats sont réalisées par le test U non paramétrique de Mann-Whitney (Logiciel Statview 5).

### 2) Résultats

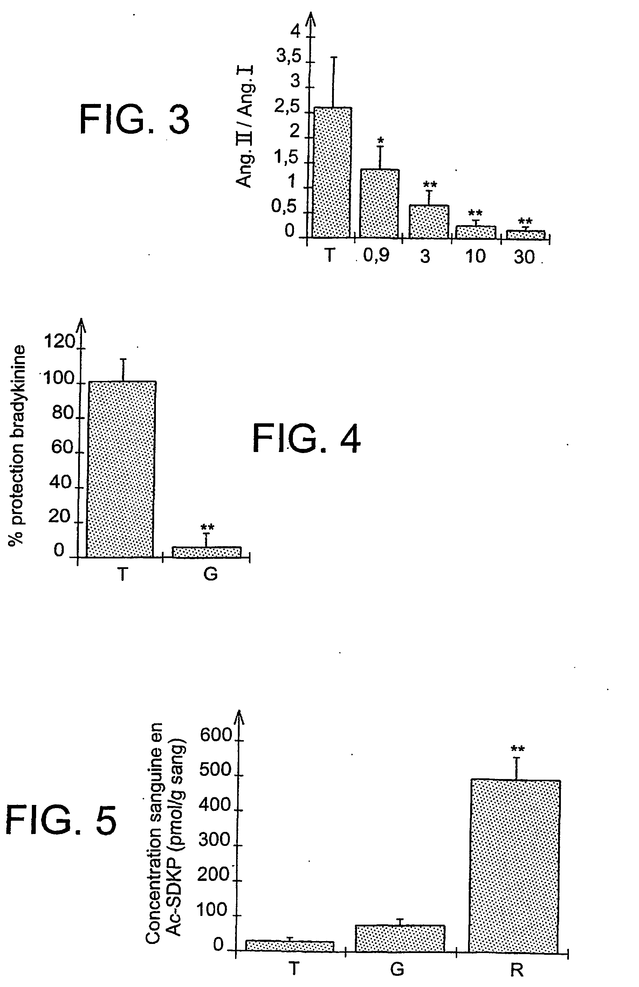

La figure 3 illustre, sous forme d'un diagramme à barres, les valeurs moyennes ± SD du rapport angiotensine II/angiotensine I obtenues chez les souris ayant reçu 0,9, 3, 10 et 30 mg de pseudo-peptide G par kg de poids corporel (barres respectivement 0,9, 3, 10 et 30) ainsi que la valeur moyenne ± SD obtenue pour ce même rapport chez les souris témoins (barre T), c'est-à-dire les souris ayant reçu 50 µl de sérum physiologique. Sur cette figure, * correspond à p<0,05, tandis que ** correspond à p<0,01, par comparaison aux souris témoins.

La figure 3 met en évidence le fait que le pseudo-peptide G est apte à inhiber *in vivo* le clivage de l'angiotensine I en angiotensine II et que cette inhibition est dose-dépendante. Ainsi, le rapport angiotensine II/angiotensine I mesuré chez les souris témoins est réduit de 50% chez les souris traitées par 0,9 mg de pseudo-peptide G par kg de poids corporel, et de 90% chez les souris traitées par 30 mg de pseudo-peptide G par kg de poids corporel.

La figure 4 illustre, sous forme d'un diagramme à barres, les pourcentages moyens ± SD de protection de la bradykinine obtenus chez les souris ayant reçu 10 mg de pseudo-peptide G par kg de poids corporel (barre G) et chez les souris témoins (barre T), c'est-à-dire, dans ce cas, les souris ayant reçu 10 mg de périndopril par kg de poids corporel. Sur cette figure, ** correspond à p<0,01 par comparaison aux souris témoins.

Comme visible sur la figure 4, le taux de protection moyen de la bradykinine par le périndopril ayant été arbitrairement fixé à 100%, ce taux de protection n'est plus que de 9,2% chez les souris ayant reçu 10 mg de pseudo-peptide G par kg de poids corporel. Ce pseudo-peptide apparaît donc n'empêcher que très modérément le clivage de la bradykinine *in vivo*.

La figure 5 illustre, également sous la forme d'un diagramme à barres, les concentrations sanguines moyennes ± SD (exprimées en pmol/g de sang) en peptide Ac-SDKP exogène marqué obtenues chez les souris ayant reçu 10 mg de pseudo-peptide G par kg de poids corporel (barre G) et chez les souris témoins (barre T), c'est-à-dire les souris ayant reçu 50 µl de sérum physiologique. Sur cette figure, ** correspond à p<0,01 par comparaison aux souris témoins.

A titre comparatif, le figure 5 montre également la concentration sanguine moyenne ± SD en peptide Ac-SDKP exogène marqué observée chez des souris traitées par 10 mg du pseudo-peptide RXP407 (décrit comme un inhibiteur sélectif du site N-terminal de l'ACE dans les références [7] et [8]) par kg de poids corporel (barre R) et en suivant un protocole opératoire identique.

Comme le montre la figure 5, le pseudo-peptide G, à la dose de 10 mg/kg de poids corporel, apparaît ne pas avoir d'effet significatif sur le clivage du peptide Ac-SDKP, alors que le RXP407 augmente le taux plasmatique de ce peptide de 16 fois par rapport à celui observé chez les souris témoins.

Ainsi, *in vivo,* le pseudo-peptide G inhibe très efficacement la conversion de l'angiotensine I en angiotensine II, sans véritablement empêcher la dégradation de la bradykinine, et encore moins celle du peptide Ac-SDKP.

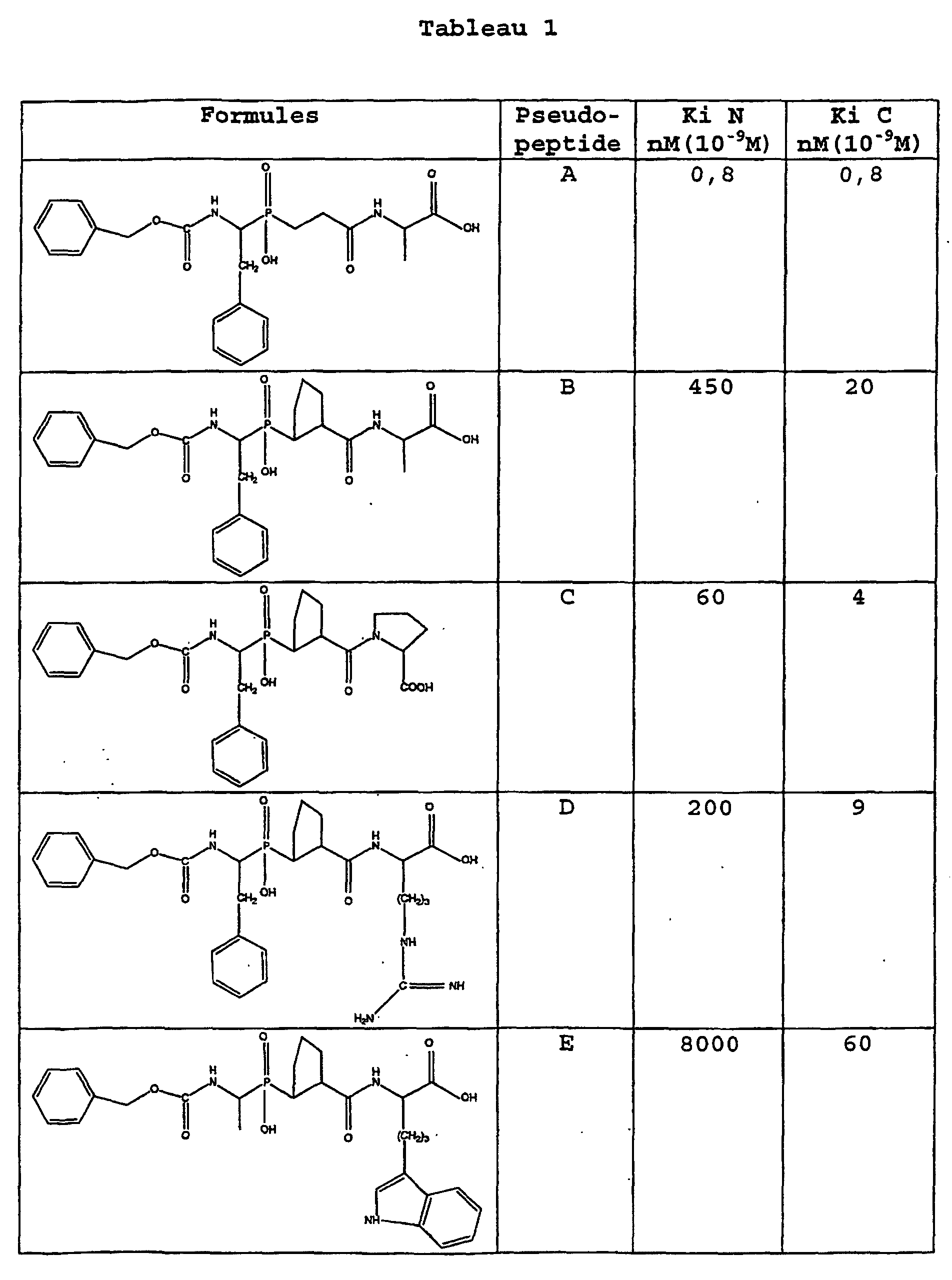

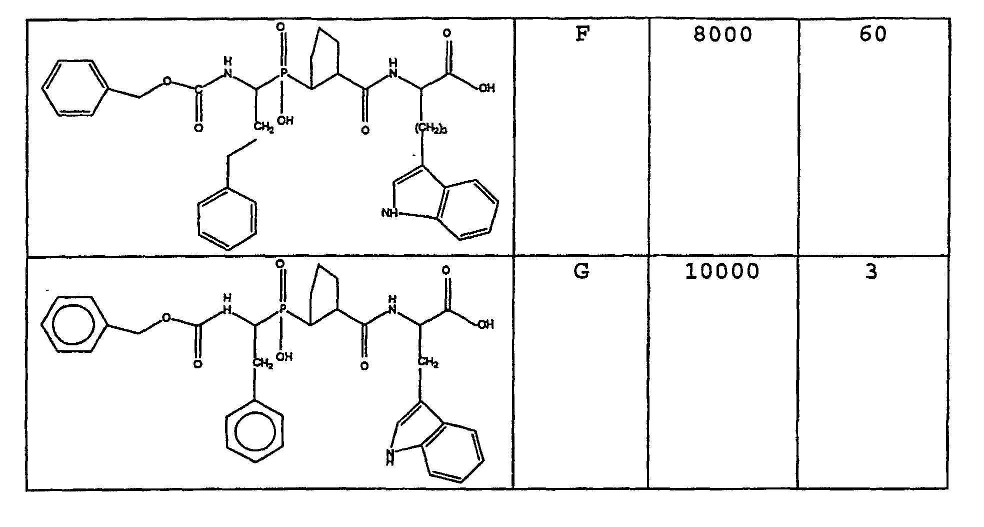

### Références

**[1]** Dzan V.J., 2001, *Hypertension* 37, 1047-1052.
**[2]** Linz W., Wiemer G., Gohlke P., Unger T., and Scholkens B.A., 1995, *Pharmacol.* Rev. 47(1), 25-49.
**[3]** Soubrier F., Alhenc-Gelas F., Hubert C., Allegrini J., John M., Tregear G., and Corvol P., 1988, *Proc. Natl. Acad. Sci. USA* 85(24), 9386-90.
**[4]** Wei L., Alhenc-Gelas F., Corvol P., and Clauser E., 1991, *J. Biol. Chem*. 266(14), 9002-8.
**[5]** Jaspard E., Wei L., and Alhenc-Gelas F. (1993) *J. Biol. Chem.* 268(13), 9496-503.
**[6]** Azizi M., Rousseau A., Ezan E., Guyene T.T., Michelet S., Grognet J.M., Lenfant M., Corvol P., and Menard J., 1996, *J. Clin. Invest.* 97(3), 839-44.
**[7]** WO-A-00/01706
**[8]** Dive V., Cotton J., Yiotakis A., Michaud A., Vassiliou S., Jiracek J., Vazeux G., Chauvet M.T., Cuniasse P., and Corvol P. (1999) *Proc. Natl. Acad. Sci. USA* 96(8), 4330-5.
**[9]** Junot C., Gonzales M.F., Ezan E., Cotton J., Vazeux G., Michaud A., Azizi M., Vassiliou S., Yiotakis A., Corvol P., and Dive V., 2001, *J. Pharmacol. Exp. Ther.* 297(2), 606-11.
**[10]** FR-A-2 676 059
**[11]** EP-A-0 725 075
**[12]** Jiracek J., Yiotakis A., Vincent B., Lecoq A., Nicolaou A., Checler F., and Dive V., Development of highly potent and selective phosphinic peptide inhibitors of zinc endopeptidase 24-15 using combinatorial chemistry., 1995, *J. Biol. Chem*. 270(37): 21701-6.
**[13]** Jiracek J., Yiotakis A., Vincent B., Checler F. and Dive V., Development of the first potent and selective inhibitor of the zinc endopeptidase neurolysin using a systematic approach based on combinatorial chemistry of phosphinic peptides, 1996, *J. Biol. Chem*. 271(32): 19606-11.
**[14]** Yiotakis A., Vassiliou S., Jiracek J., and Dive V., Protection of the hydroxy-phosphinyl function of phosphinic dipeptides by adamatyl. Application to the solid-phase synthesis of phosphinic peptides, 1996, *J. Org. Chem*. 61 : 6601-6605.
**[15]** Vassiliou S., Mucha A., Cuniasse P., Georgiadis D., Lucet-Levannier K., Beau F., Kannan R., Murphy G., Knauper V., Rio MC., Basset P., Yiotakis A., and Dive V., Phosphinic pseudo-tripeptides as potent inhibitors of matrix metalloproteinases : a structure-activity study, 1999, *J. Med. Chem.* 42(14): 2610-20.
**[16]** Georgiadis D., Vazeux G., Llorens-Cortes C., Yiotakis A., and Dive V., Potent and selective inhibition of zinc aminopeptidase A (EC 3.4.11.7, APA) by glutamyl aminophosphinic peptides : importance of glutamyl aminophosphinic residue in the P1 position, 2000, *Biochemistry* 39(5): 1152-5.
**[17]** Protective groups in Organic Synthesis, Second Edition, T.W. Green et P.G.M. Wuts, John Wyley & Sons, Inc., 309-315.
**[18]** Baylis E.K., Campbell C.D., and Dingwall J.G., 1984, *J. Chem. Soc. Perkin. Trans. I*, 2845.
**[19]** Villieras J., Rambaud W.H., and Graff M., 1986, *Synth. Commun*. 16, 149.
**[20]** Chen H., Noble F., Roques P., and Fournie-Zaluski M.C., 2001, *J. Med. Chem.* 44, 3523-3530.
**[21]** FR-A-2 781 230.
**[22]** Demange L. and Dugaur C, 2001, Tetrahedron Letters 42, 6295-6297.
**[23]** EP-A-0 361 341.
**[24]** US-A-5,476,847.

## Revendications

1. Utilisation d'au moins un dérivé de pseudo-peptide phosphinique comportant la séquence d'acides aminés de formule (I) suivante : 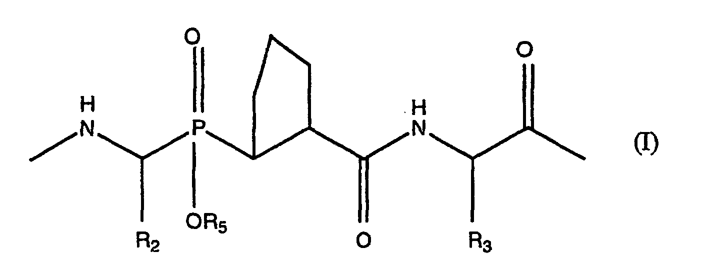 dans laquelle :
- R₂ et R₃, qui sont identiques ou différents, représentent la chaîne latérale d'un acide aminé naturel ou non naturel, l'ensemble : 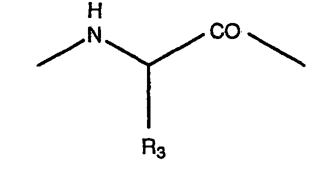 pouvant aussi former le résidu Pro, et
- R₅ représente un atome d'hydrogène, un contre-ion acceptable du point de vue pharmacologique, ou un groupe capable de former un ester phosphinique hydrolysable *in vivo* ;
pour la fabrication d'un médicament inhibant sélectivement le site C-terminal de l'enzyme de conversion de l'angiotensine I.

2. Utilisation d'un dérivé de pseudo-peptide phosphinique répondant à la formule (II) suivante : 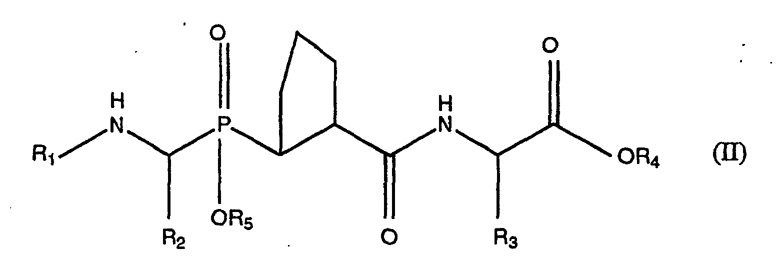 dans laquelle :
- R₁ représente un groupe protecteur d'une fonction amine, ou un acide aminé ou un peptide protégé par un groupe protecteur d'une fonction amine,
- R₂ et R₃, qui peuvent être identiques ou différents, représentent la chaîne latérale d'un acide aminé naturel ou non naturel, l'ensemble : 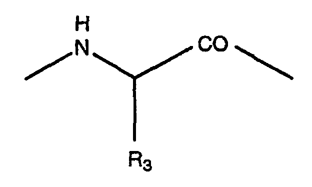 pouvant aussi former le résidu Pro,
- R₄ représente un atome d'hydrogène ou un contre-ion acceptable du point de vue pharmacologique, et
- R₅ représente un atome d'hydrogène, un contre-ion acceptable du point de vue pharmacologique, ou un groupe capable de former un ester phosphinique hydrolysable *in vivo* ;
pour la fabrication d'un médicament inhibant sélectivement le site C-terminal de l'enzyme de conversion de l'angiotensine I.

3. Utilisation selon la revendication 2, dans laquelle R₁ représente un groupe protecteur d'une fonction amine choisi parmi les groupes acétyle et benzyloxycarbonyle.

4. Utilisation selon l'une quelconque des revendications 1 à 3, dans laquelle R₂ représente le groupe benzyle, méthyle ou phénéthyle.

5. Utilisation selon l'une quelconque des revendications 1 à 4, dans laquelle R₃ représente la chaîne latérale de l'alanine, de l'arginine ou du tryptophane.

6. Utilisation selon l'une quelconque des revendications 1 à 4, dans laquelle l'ensemble -NH-CH(R₃)-CO- forme le résidu Pro :

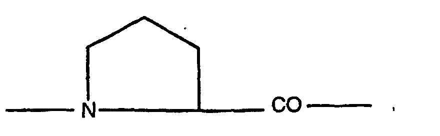

7. Utilisation selon l'une quelconque des revendications 1 à 6, dans laquelle R₄ et/ou R₅. représentent un atome d'hydrogène.

8. Utilisation selon la revendication 2, dans laquelle le dérivé de pseudo-peptide phosphinique répond à la formule :

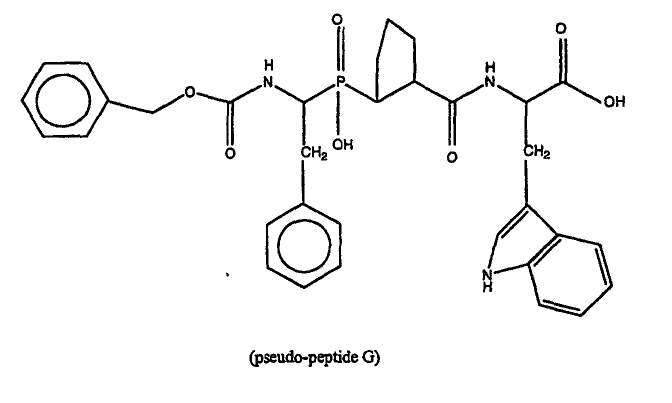

9. Dérivé de pseudo-peptide phosphinique comportant la séquence d'acides aminés de formule (I) suivante : 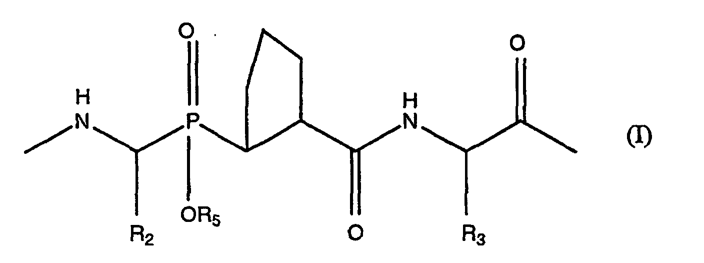 dans laquelle :
- R₂ représente la chaîne latérale d'un acide aminé naturel ou non naturel,
- l'ensemble : 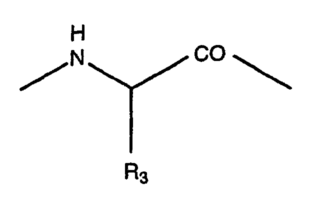 forme le résidu Pro : 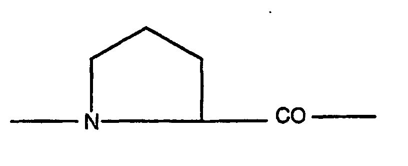
- R₄ représente un atome d'hydrogène ou un contre-ion acceptable du point de vue pharmacologique,
- R₅ représente un atome d'hydrogène, un contre-ion acceptable du point de vue pharmacologique, ou un groupe capable de former un ester phosphinique hydrolysable *in vivo.*

10. Dérivé de pseudo-peptide phosphinique répondant à la formule (II) suivante : 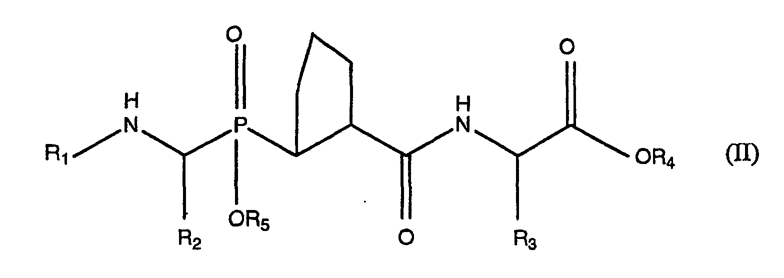 dans laquelle :
- R₁ représente un groupe protecteur d'une fonction amine, ou un acide aminé ou un peptide protégé par un groupe protecteur d'une fonction amine,
- R₂ représente la chaîne latérale d'un acide aminé naturel ou non naturel,
- l'ensemble : 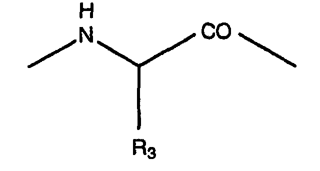 forme le résidu Pro : 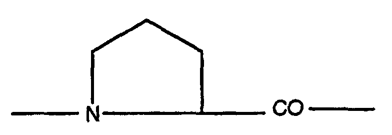
- R₅ représente un atome d'hydrogène, un contre-ion acceptable du point de vue pharmacologique, ou un groupe capable de former un ester phosphinique hydrolysable *in vivo*.

11. Dérivé de pseudo-peptide phosphinique de formule :

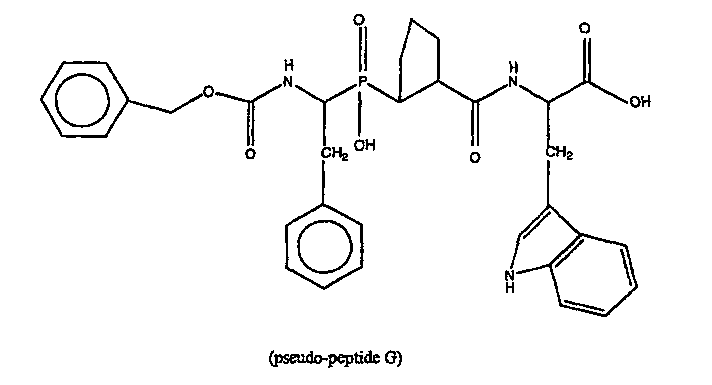

12. Composition pharmaceutique comprenant au moins un dérivé de pseudo-peptide phosphinique selon l'une quelconque des revendications 9 à 11.

13. Composition pharmaceutique, dans laquelle le dérivé pseudo-peptidique phosphinique répond à la formule :

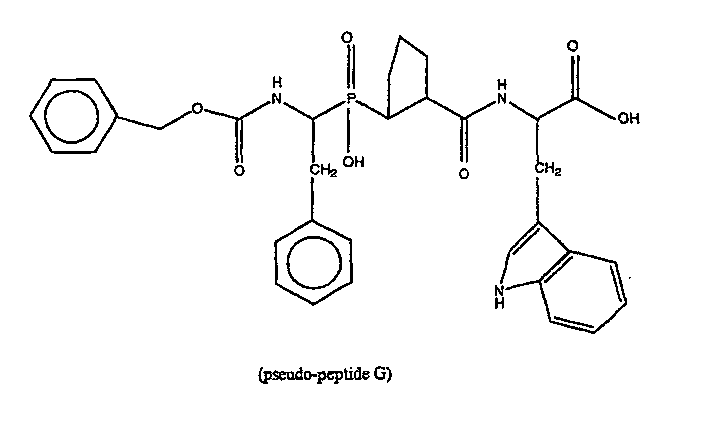

14. Procédé de préparation d'un pseudo-peptide de formule : 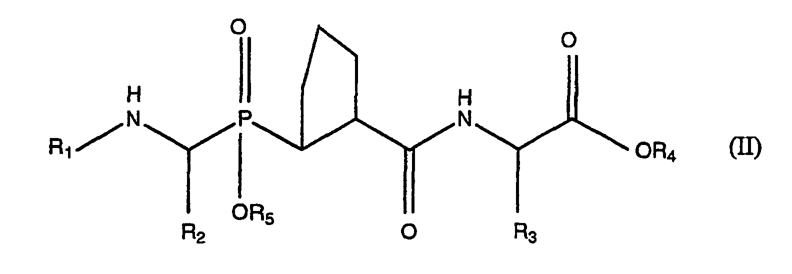 dans laquelle :
- R₁ représente un groupe protecteur d'une fonction amine, ou un acide aminé ou un peptide protégé par un groupe protecteur d'une fonction amine,
- R₂ et R₃, qui peuvent être identiques ou différents, représentent la chaîne latérale d'un acide aminé naturel ou non naturel, l'ensemble : 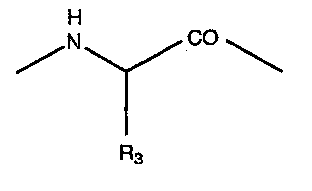 pouvant aussi former le résidu Pro, et
- R₄ et R₅ représentent un atome d'hydrogène ; qui comprend les étapes suivantes :
1) faire réagir un composé de formule (III): 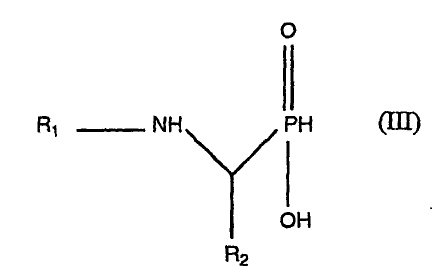 dans laquelle R₁ et R₂ sont tels que définis ci-dessus, avec le composé de formule (IV) : 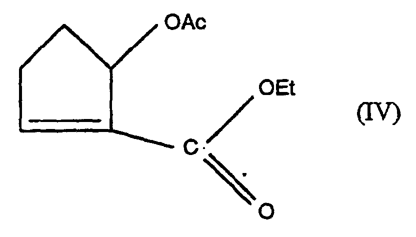 dans laquelle AC représente le groupe acétyle et Et représente le groupe éthyle, pour obtenir le composé de formule (V) : 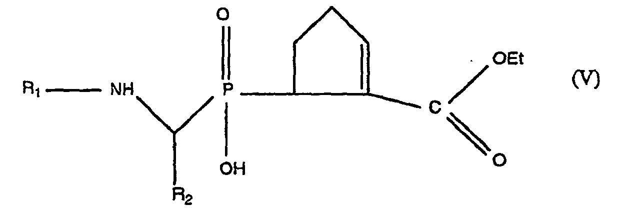
2) transformer le composé (V) en composé (VI) par réaction du composé (V) avec du borohydrure de sodium : 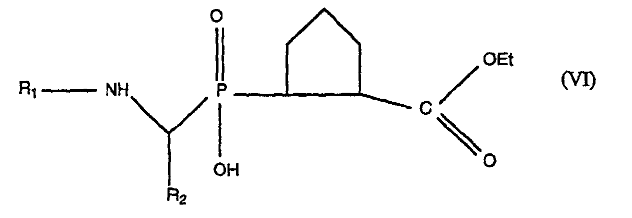
3) protéger le groupe hydroxyle du composé (VI) par un groupe protecteur R₅, par exemple le groupe adamantyle Ad, pour obtenir le composé de formule (VII) : 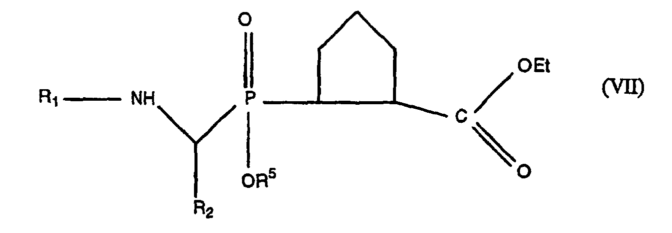
4) saponifier le composé (VII) pour obtenir le composé de formule (VIII) : 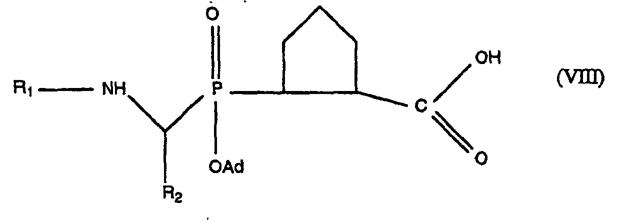
5) coupler le composé de formule (VIII) avec l'acide aminé de formule (iX) ou (X) : 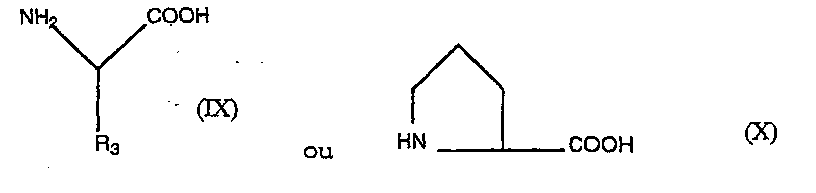 dans laquelle R₃ est tel que défini ci-dessus, et
6) éliminer le groupe protecteur Ad.

15. Procédé selon la revendication 14, dans lequel l'étape 5) de couplage peptidique est réalisée par synthèse peptidique sur phase solide en utilisant comme phase solide une résine substituée par l'acide aminé de formule (IX) ou (X).

16. Procédé de préparation d'un pseudo-peptide de formule : 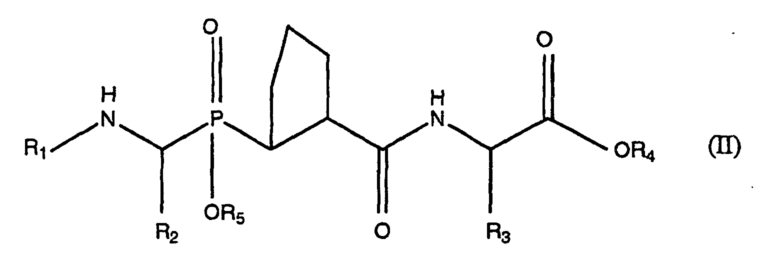 dans laquelle :
- R₁ représente un groupe protecteur d'une fonction amine, ou un acide aminé ou un peptide protégé par un groupe protecteur d'une fonction amine,
- R₂ et R₃, qui peuvent être identiques ou différents, représentent la chaîne latérale d'un acide aminé naturel ou non naturel, l'ensemble : 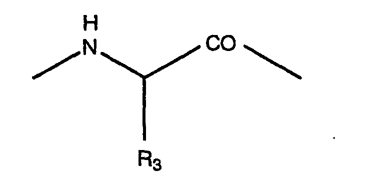 pouvant aussi former le résidu Pro,
- R₄ représente un atome d'hydrogène, et
- R₅ représente un groupe capable de former un ester phosphinique hydrolysable *in vivo* ;
dans lequel on estérifie la fonction phosphinique du pseudo-peptide obtenu par le procédé de la revendication 14 ou 15, par couplage avec un alcool de formule R₅OH ou par réaction avec un halogénure de formule R₅X où X représente un atome d'halogène.

17. Composé de formule (VIII) : 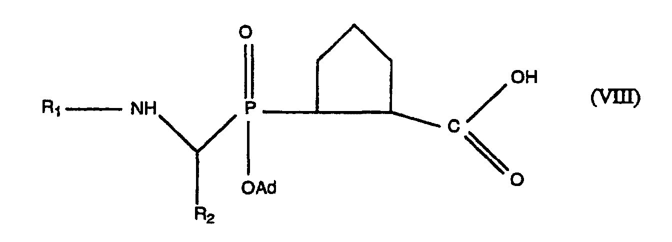 dans laquelle :
- R₁ représente un groupe protecteur d'une fonction amine ou un acide aminé ou un peptide protégé par un groupe protecteur d'une fonction amine, et
- R₂ représente la chaîne latérale d'un acide aminé naturel ou non naturel.

## Patentansprüche

1. Verwendung mindestens eines Phosphinsäurepseudopeptid-Derivats, das die folgende Aminosäure-Sequenz der Formel (I) umfasst: 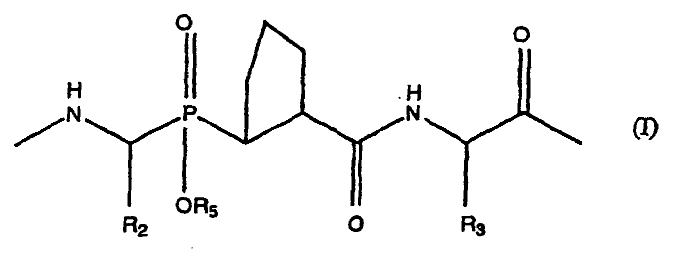 worin:
- R₂ und R₃, die gleich oder verschieden sind, die Seitenkette einer natürlichen oder nicht-natürlichen Aminosäure darstellen, wobei die Einheit 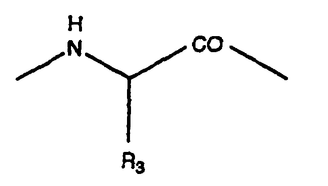 außerdem den Pro-Rest bilden kann, und
- R₅ ein Wasserstoffatom, ein pharmakologisch akzeptables Gegenion oder eine Gruppe darstellt, die in vivo einen hydrolysierbaren Phosphinsäureester bilden kann;
zur Herstellung eines Arzneimittels, das die C-terminale Stelle des Enzyms zur Umwandlung von Angiotensin I selektiv blockiert.

2. Verwendung eines Phosphinsäurepseudopeptid-Derivats der folgenden Formel (II): 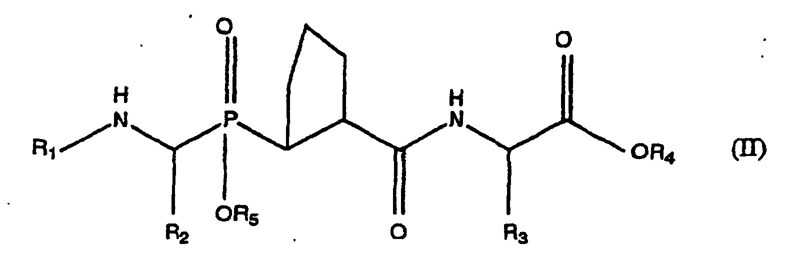 worin:
- R₁ eine Schutzgruppe für eine Aminfunktion oder eine Aminosäure oder ein durch eine Schutzgruppe für eine Aminfunktion geschütztes Peptid darstellt,
- R₂ und R₃, die gleich oder verschieden sein können, die Seitenkette einer natürlichen oder nicht-natürlichen Aminosäure darstellen, wobei die Einheit 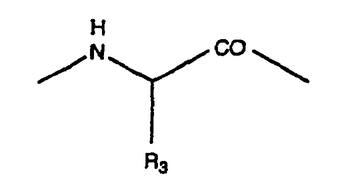 außerdem den Pro-Rest bilden kann,
- R₄ ein Wasserstoffatom oder ein pharmakologisch akzeptables Gegenion darstellt und
R₅ ein Wasserstoffatom, ein pharmakologisch akzeptables Gegenion oder eine Gruppe darstellt, die in vivo einen hydrolysierbaren Phosphinsäureester bilden kann;
zur Herstellung eines Arzneimittels, das die C-terminale Stelle des Enzyms zur Umwandlung von Angiotensin I selektiv blockiert.

3. Verwendung nach Anspruch 2, bei der R₁ eine Schutzgruppe für eine Amin-Funktion, ausgewähtt unter den Acetyl- und Benzyloxycarbonylgruppen, darstellt.

4. Verwendung nach einem der Ansprüche 1 bis 3, bei der R₂ die Benzyl-, Methyl- oder Phenethyl-Gruppe darstellt.

5. Verwendung nach einem der Ansprüche 1 bis 4, bei der R₃ die Seitenkette von Alanin, Arginin oder Tryptophan darstellt.

6. Verwendung nach einem der Ansprüche 1 bis 4, bei der die Einheit -NH-CH(R₃)-CO- den Pro-Rest bildet

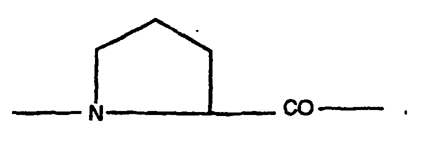

7. Verwendung nach einem der Ansprüche 1 bis 6, bei der R₄ und/oder R₅ ein Wasserstoffatom darstellt (darstellen).

8. Verwendung nach Anspruch 2, bei der das Phosphinsäurepseudopeptid-Derivat der Formel entspricht:

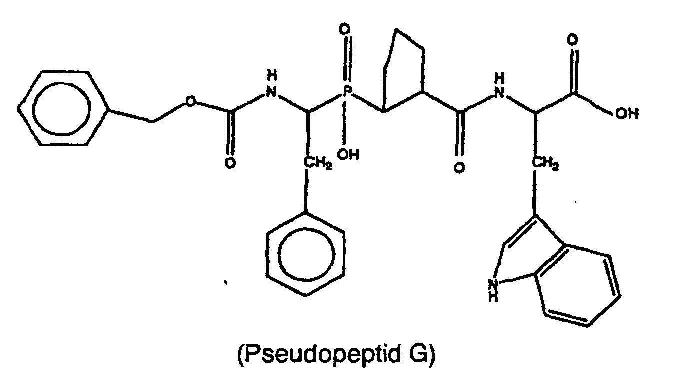

9. Phosphinsäurepseudopeptid-Derivat, das die Aminosäure-Sequenz der folgenden Formel (I) umfasst: 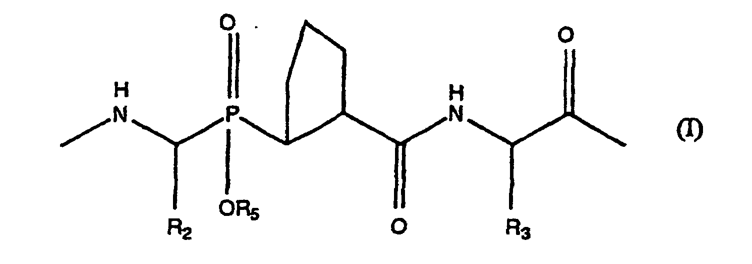 worin:
- R₂ die Seitenkette einer natürlichen oder nicht-natürlichen Aminosäure darstellt,
- die Einheit 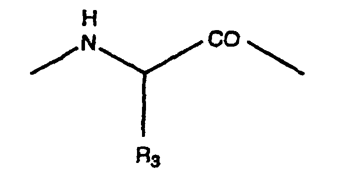 den Pro-Rest bildet: 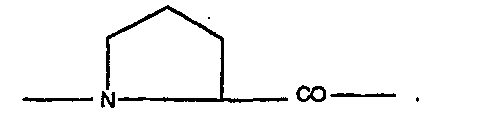
- R₄ ein Wasserstoffatom oder ein pharmakologisch akzeptables Gegenion darstellt,
- R₅ ein Wasserstoffatom, ein pharmakologisch akzeptables Gegenion oder eine Gruppe darstellt, die in vivo einen hydrolysierbaren Phosphinsäureester bilden kann.

10. Phosphinsäurepseudopeptid-Derivat der folgenden Formel (II): 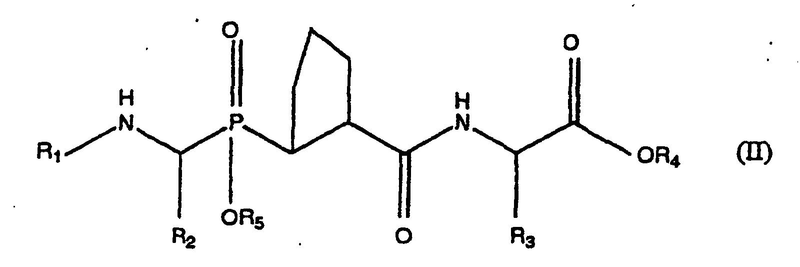 worin:
- R₁ eine Schutzgruppe für eine Aminfunktion oder eine Aminosäure oder ein durch eine Schutzgruppe für eine Aminfunktion geschütztes Peptid darstellt,
- R₂ die Seitenkette einer natürlichen oder nicht-natürlichen Aminosäure darstellt,
- die Einheit 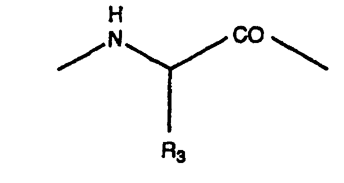 den Pro-Rest bildet: 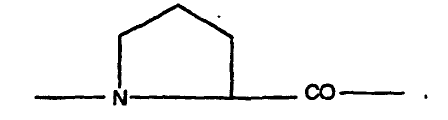
- R₅ ein Wasserstoffatom, ein pharmakologisch akzeptables Gegenion oder eine Gruppe darstellt, die in vivo einen hydrolysierbaren Phosphinsäureester bilden kann.

11. Phosphinsäurepseudopeptid-Derivat der Formel:

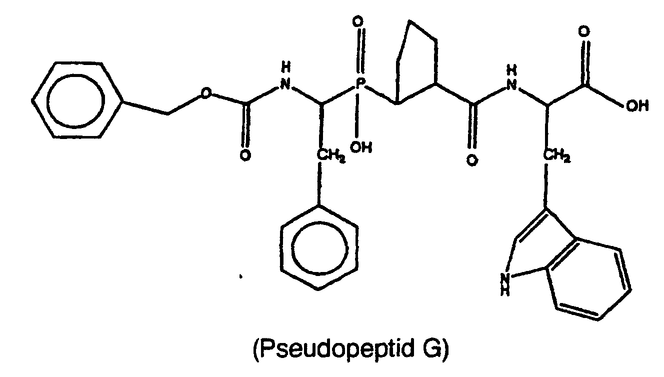

12. Pharmazeutische Zusammensetzung, die mindestens ein Phosphinsäurepseudopeptid-Derivat nach einem der Ansprüche 9 bis 11 umfasst.

13. Pharmazeutische Zusammensetzung, in der das Phosphinsäurepseudopeptid-Derivat der Formel entspricht:

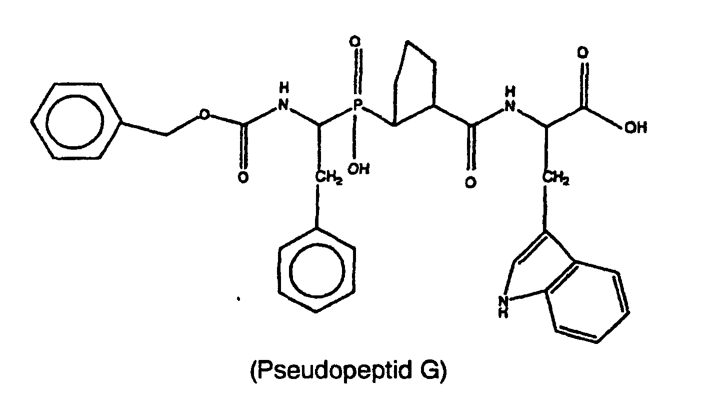

14. Verfahren zur Herstellung eines Pseudopeptids der Formel: 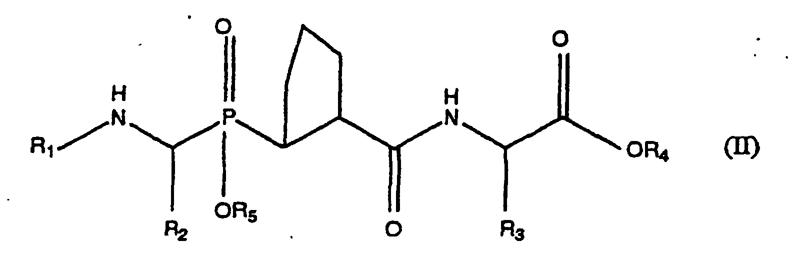 worin:
- R₁ eine Schutzgruppe für eine Amin-Funktion oder eine Aminosäure oder ein durch eine Schutzgruppe für eine Amin-Funktion geschütztes Peptid darstellt,
- R₂ und R₃, die gleich oder verschieden sein können, die Seitenkette einer natürlichen oder nicht-natürlichen Aminosäure darstellen, wobei die Einheit: 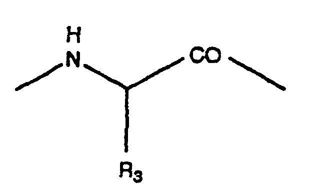 außerdem den Pro-Rest bilden kann, und
- R₄ und R₅ ein Wasserstoffatom darstellen;
wobei das Verfahren die folgenden Stufen umfasst:
1) Reagierenlassen einer Verbindung der Formel (III): 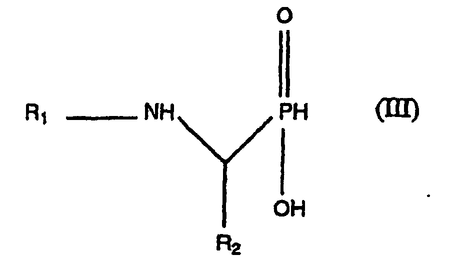 in der R₁ und R₂ wie oben definiert sind,
mit einer Verbindung der Formel (IV): 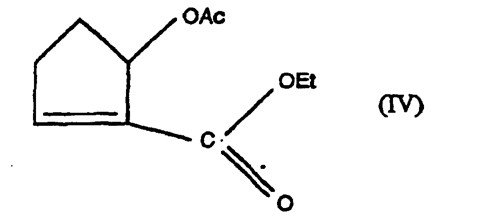 in der Ac die Acetyl-Gruppe darstellt und Et die Ethyl-Gruppe darstellt,
zur Herstellung der Verbindung der Formel (V): 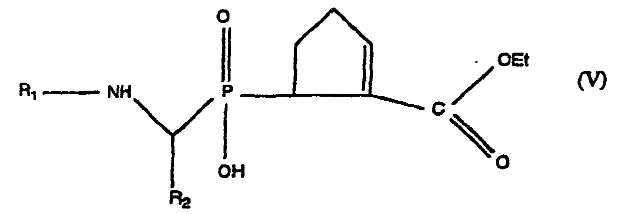
2) Umwandlung der Verbindung (V) in eine Verbindung (VI) durch Umsetzung der Verbindung (V) mit Natriumborhydrid: 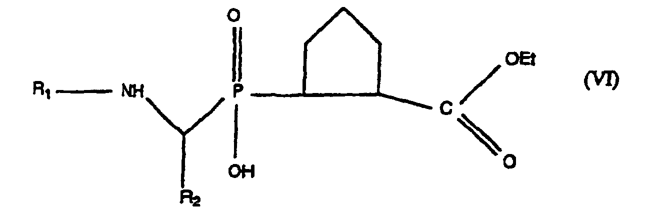
3) Schützen der Hydroxylgruppe der Verbindung (VI) mit einer Schutzgruppe R₅, beispielsweise mit der Adamantyl-Gruppe Ad, zur Herstellung der Verbindung der Formel (VII): 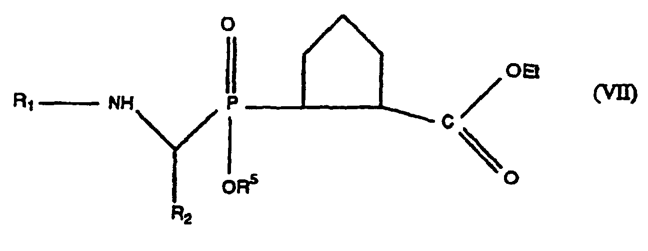
4) Verseifen der Verbindung (VII) zur Herstellung der Verbindung der Formel (VIII): 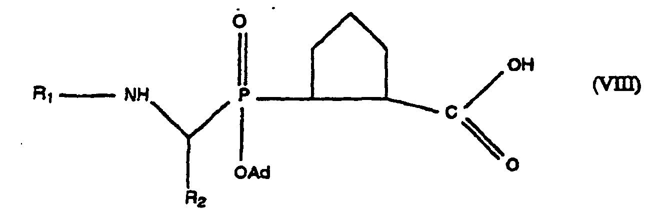
5) Kuppeln der Verbindung der Formel (VIII) mit einer Aminosäure der Formel (IX) oder (X): 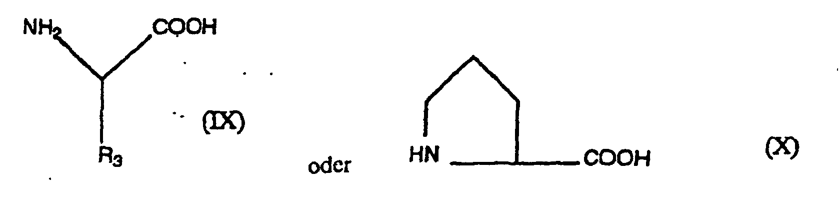 worin R₃ wie oben definiert ist, und
6) Eliminieren der Schutzgruppe Ad.

15. Verfahren nach Anspruch 14, bei dem die Stufe (5) der Peptid-Kupplung durchgeführt wird durch Peptid-Synthese in fester Phase unter Verwendung eines Harzes, das durch die Aminosäure der Formel (IX) oder (X) substituiert ist, als feste Phase.

16. Verfahren zur Herstellung eines Pseudopeptids der Formel: 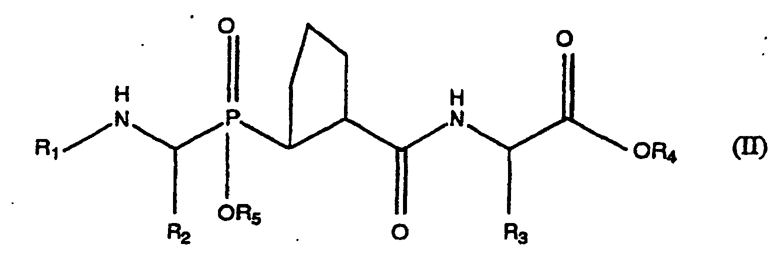 worin:
- R₁ eine Schutzgruppe für eine Aminfunktion oder eine Aminosäure oder ein durch eine Schutzgruppe für eine Aminfunktion geschütztes Peptid darstellt,
- R₂ und R₃, die gleich oder verschieden sein können, die Seitenkette einer natürlichen oder nicht-natürlichen Aminosäure darstellen,
wobei die Einheit 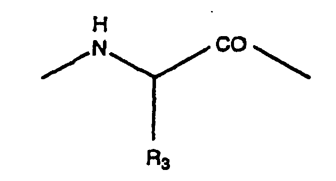 außerdem den Pro-Rest bilden kann,
- R₄ ein Wasserstoffatom darstellt und
R₅ eine Gruppe darstellt, die in vivo einen hydrolysierbaren Phosphinsäureester bilden kann;
wobei man in dem Verfahren die Phosphinsäure-Funktion des nach dem Verfahren nach Anspruch 14 oder 15 erhaltenen Pseudopeptids verestert durch Kuppeln mit einem Alkohol der Formel R₅OH oder durch Umsetzung mit einem Halogenid der Formel R₅X, worin X für ein Halogenatom steht.

17. Verbindung der Formel (VIII): 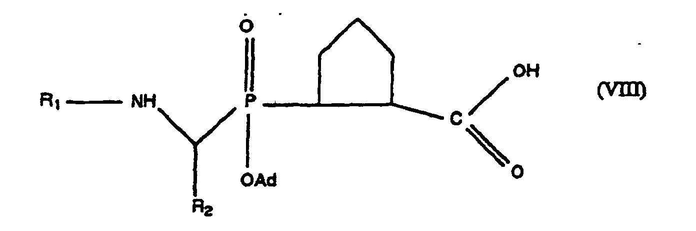 worin:
- R₁ eine Schutzgruppe für eine Amin-Funktion oder eine Aminosäure oder ein durch eine Schutzgruppe für eine Amin-Funktion geschütztes Peptid steht und
- R₂ die Seitenkette einer natürlichen oder nicht-natürlichen Aminosäure darstellt.

## Claims

1. Use of at least one phosphinic pseudopeptide derivative comprising the amino acid sequence of formula (I) below: 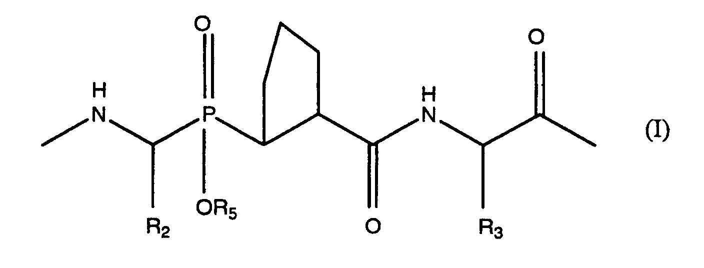 in which:
- R₂ and R₃, which are identical or different, represent the side chain of a natural or unnatural amino acid, the sequence: 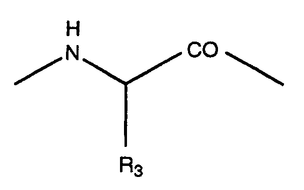 also possibly forming the Pro (proline) residue, and
- R₅ represents a hydrogen atom, a pharmacologically acceptable counterion, or a group capable of forming an in vivo hydrolysable phosphinic ester; for the manufacture of a medicinal product capable of selectively inhibiting the C-terminal site of angiotensin I converting enzyme.

2. Use of a phosphinic pseudopeptide derivative corresponding to formula (II) below: 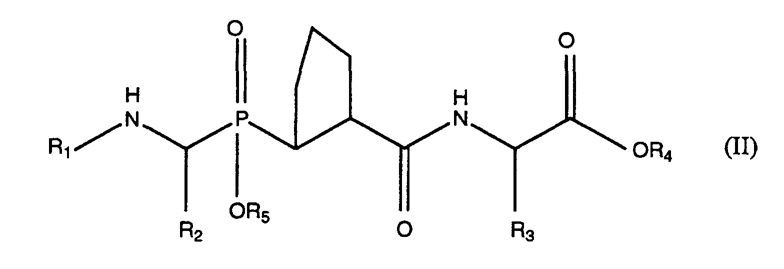 in which:
- R₁ represents a protecting group for an amine function, or an amino acid or a peptide protected with a protecting group for an amine function,
- R₂ and R₃, which may be identical or different, represent the side chain of a natural or unnatural amino acid, the sequence: 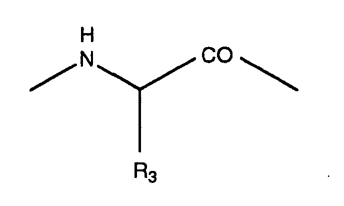 also possibly forming the Pro residue,
- R₄ represents a hydrogen atom or a pharmacologically acceptable counterion, and
- R₅ represents a hydrogen atom, a pharmacologically acceptable counterion, or a group capable of forming an *in vivo* hydrolysable phosphinic ester;
for the manufacture of a medicinal product capable of selectively inhibiting the C-terminal site of angiotensin I converting enzyme.

3. Use according to Claim 2, in which R₁ represents a protecting group for an amine function chosen from acetyl and benzyloxycarbonyl groups.

4. Use according to any one of Claims 1 to 3, in which R₂ represents the benzyl, methyl or phenylethyl group.

5. Use according to any one of Claims 1 to 4, in which R₃ represents the side chain of alanine, arginine or tryptophan.

6. Use according to any one of Claims 1 to 4, in which the sequence -NH-CH(R₃)-CO- forms the Pro residue:

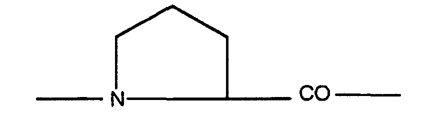

7. Use according to any one of Claims 1 to 6, in which R₄ and/or R₅ represent(s) a hydrogen atom.

8. Use according to Claim 2, in which the phosphinic pseudopeptide derivative corresponds to the formula:

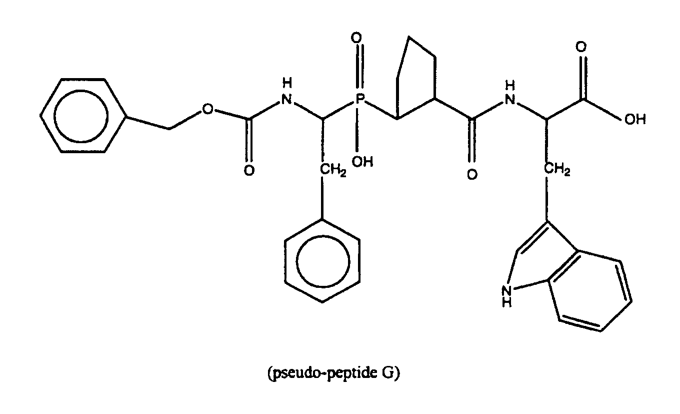

9. Phosphinic pseudopeptide derivative comprising the amino acid sequence of formula (I) below: 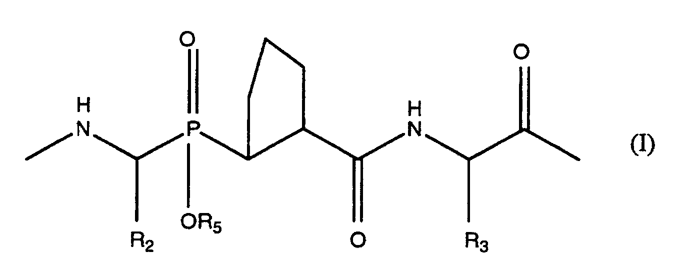 in which:
- R₂ represents the side chain of a natural or unnatural amino acid,
- the sequence: 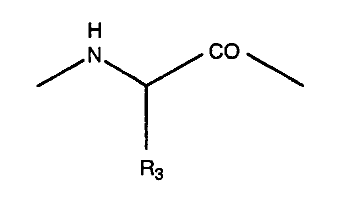 forms the Pro residue: 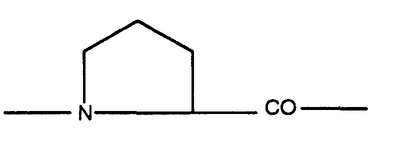
- R₄ represents a hydrogen atom or a pharmacologically acceptable counterion, and
- R₅ represents a hydrogen atom, a pharmacologically acceptable counterion, or a group capable of forming an *in vivo* hydrolysable phosphinic ester.

10. Phosphinic pseudopeptide derivative corresponding to formula (II) below: 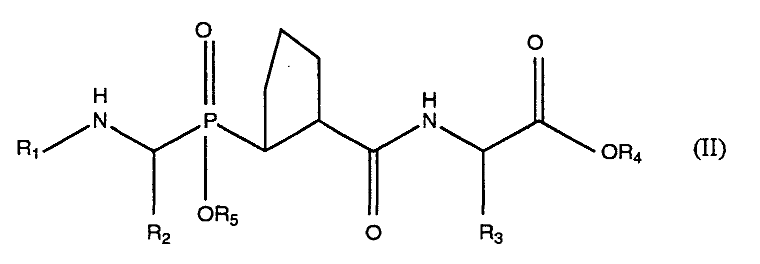 in which:
- R₁ represents a protecting group for an amine function, or an amino acid or a peptide protected with a protecting group for an amine function,
- R₂ represents the side chain of a natural or unnatural amino acid,
- the sequence: 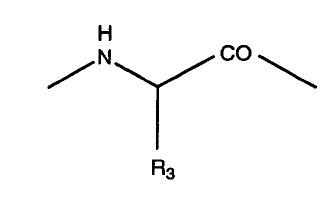 forms the Pro residue: 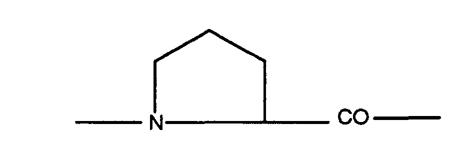
- R₅ represents a hydrogen atom, a pharmacologically acceptable counterion, or a group capable of forming an *in vivo* hydrolysable phosphinic ester.

11. Phosphinic pseudopeptide derivative of formula:

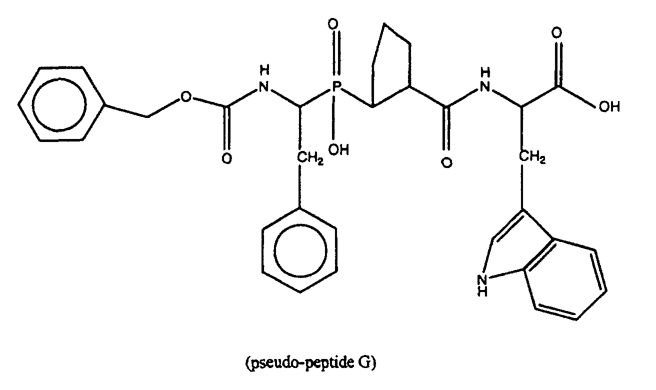

12. Pharmaceutical composition comprising at least one phosphinic pseudopeptide derivative according to any one of Claims 9 to 11.

13. Pharmaceutical composition, in which the phosphinic pseudopeptide derivative corresponds to the formula:

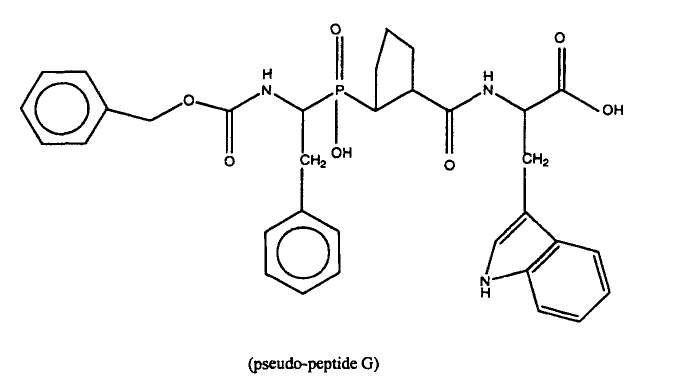

14. Process for preparing a pseudopeptide of formula: 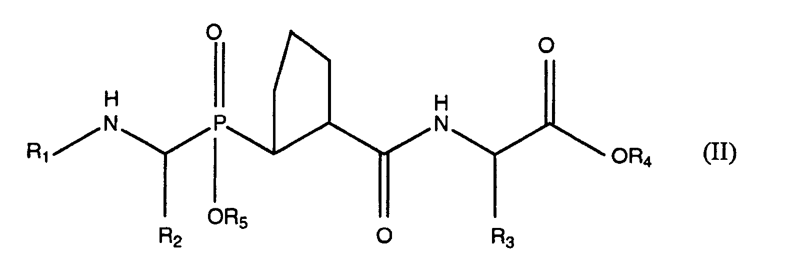 in which:
- R₁ represents a protecting group for an amine function, or an amino acid or a peptide protected with a protecting group for an amine function,
- R₂ and R₃, which may be identical or different, represent the side chain of a natural or unnatural amino acid, the sequence: 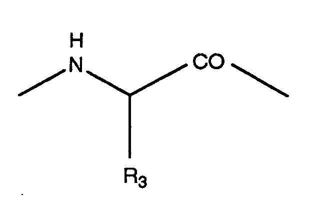 also possibly forming the Pro residue, and
- R₄ and R₅ represent a hydrogen atom;
which comprises the following steps:
1) reacting a compound of formula (III): 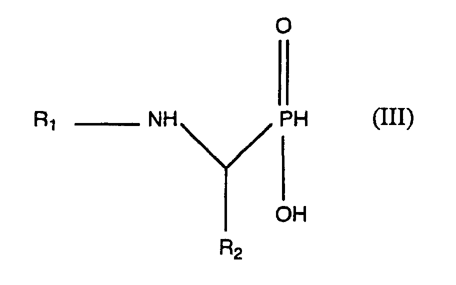 in which R₁ and R₂ are as defined above, with the compound of formula (IV): 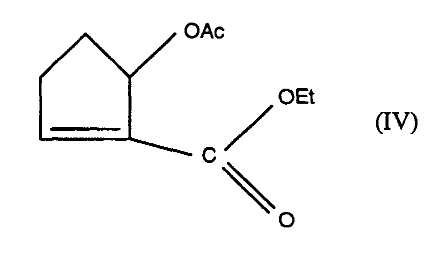 in which Ac represents the acetyl group and Et represents the ethyl group, to obtain the compound of formula (V): 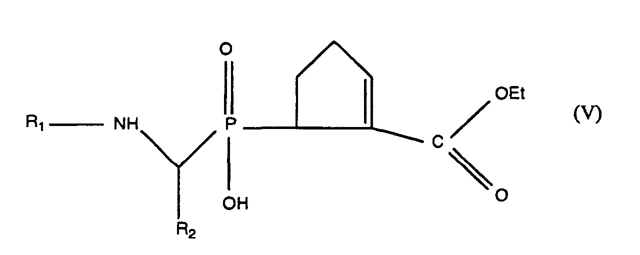
2) converting compound (V) into compound (VI) by reacting compound (V) with sodium borohydride: 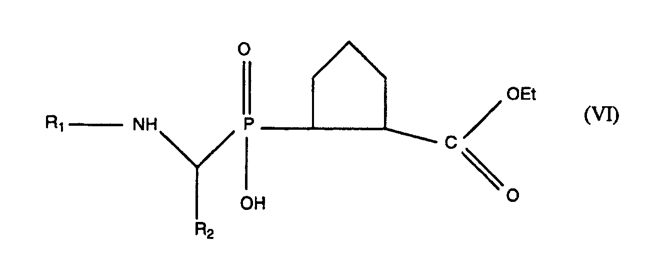
3) protecting the hydroxyl group of compound (VI) with a protecting group R₅, for example the adamantyl group Ad, to give the compound of formula (VII): 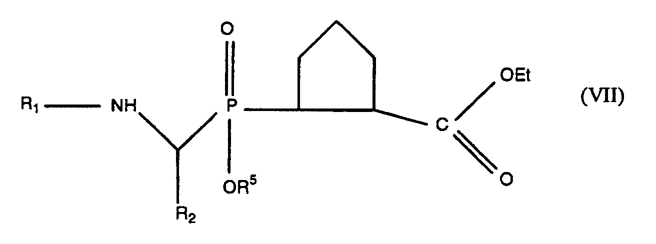
4) saponifying compound (VII) to give the compound of formula (VIII): 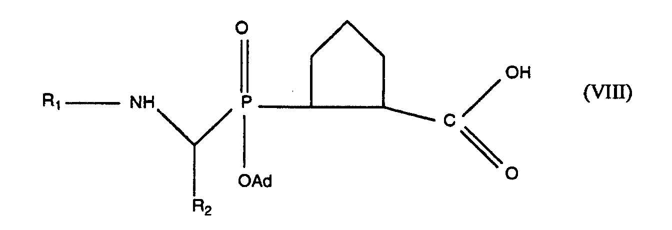
5) coupling the compound of formula (VIII) with the amino acid of formula (IX) or (X): 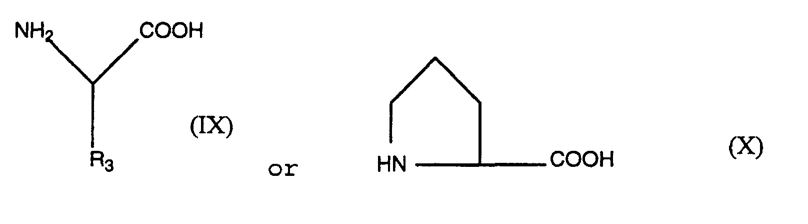 in which R₃ is as defined above, and
6) removing the protecting group Ad.

15. Process according to Claim 14, in which the peptide coupling step 5) is performed via solid-phase peptide synthesis using as solid phase a resin substituted with the amino acid of formula (IX) or (X).

16. Process for preparing a pseudopeptide of formula: 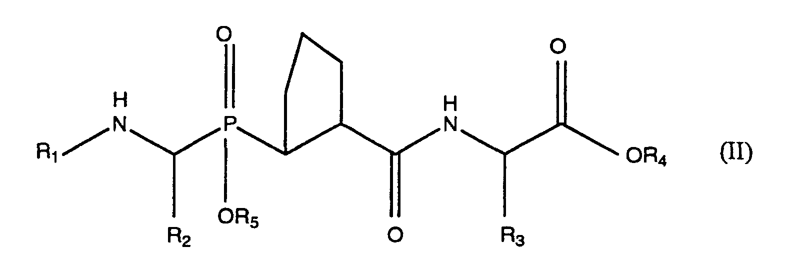 in which:
- R₁ represents a protecting group for an amine function, or an amino acid or a peptide protected with a protecting group for an amine function,
- R₂ and R₃, which may be identical or different, represent the side chain of a natural or unnatural amino acid, the sequence: 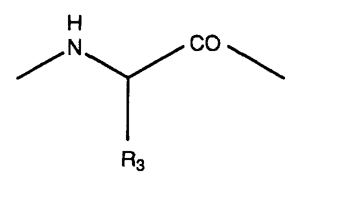 also possibly forming the Pro residue,
- R₄ represents a hydrogen atom, and
- R₅ represents a group capable of forming an *in vivo* hydrolysable phosphinic ester;
in which the phosphinic function of the pseudopeptide obtained via the process of Claim 14 or 15 is esterified by coupling with an alcohol of formula R₅OH or by reaction with a halide of formula R₅X in which X represents a halogen atom.

17. Compound of formula (VIII): 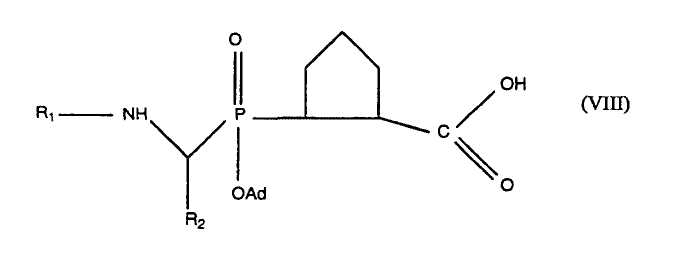 in which:
- R₁ represents a protecting group for an amine function or an amino acid or a peptide protected with an amine function, and
- R₂ represents the side chain of a natural or unnatural amino acid.
